# FASCICULE DE BREVET EUROPEEN

(11) **EP 0 511 116 B1**
(45) Date de publication et mention de la délivrance du brevet: **16.06.2004**
(21) Numéro de dépôt: 92401185.1
(22) Date de dépôt: 24.04.1992
(51) Int. Cl.: C07K 14/47, C07K 16/00, G01N 33/564

(54) **Antigènes reconnus par des anticorps de la polyarthrite rhumatoide, leur préparation et leurs applications**
Antigene, die von Polyarthritis Rheumatoide Antikörpern erkannt werden, ihre Herstellung und ihre Anwendungen
Antigens recognized by polyarthritis rheumatoid antibodies, preparation and applications

(30) Priorité: 26.04.1991 FR 9104983; 24.09.1991 FR 9111727
(43) Date de publication de la demande: 28.10.1992
(73) Titulaire: CLONATEC S.A., F-75580 Paris Cédex 12 (FR)
(72) Inventeur: Serre, Guy, F-31100 Toulouse (FR); Sommé, Gérard, F-91440 Bures Sur Yvette (FR); Vincent, Christian, F-31660 Lauzerville (FR)
(74) Mandataire: Orès, Bernard

## Description

La présente Invention est relative à l'utilisation de la filaggrine humaine, et d'antigènes apparentés à celle-ci, pour le diagnostic ou le traitement de la polyarthrite rhumatoïde (PR).

La PR est le plus fréquent des rhumatismes inflammatoires chroniques. Elle atteint environ 2 % de la population des pays développés.

La PR est évolutive, invalidante et pose des problèmes de diagnostic difficiles dans ses formes de début, alors même qu'elle appelle des traitements spécifiques. En dehors des signes cliniques et radiologiques, le rhumatologue ne dispose actuellement que de tests biologiques peu spécifiques pour son diagnostic. Les plus utilisés d'entre eux sont le test de Waaler-Rose et le test au latex, qui détectent le facteur rhumatoïde dans 7 PR sur 10. Cependant, la spécificité de ces tests est très mauvaise puisque ces facteurs sont détectés dans la plupart des autres pathologies autoimmunes et même chez des individus sains.

La présence d'autoanticorps dirigés contre des composants cellulaires est la caractéristique générale des maladies autoimmunes telles que la PR, le lupus érythémateux disséminé, la sclérodermie ou la polymyosite. Parmi les nombreux types d'autoanticorps identifiés dans ces maladies, ceux spécifiquement présents chez les malades atteints de PR et réagissant avec un antigène épithélial oesophagien ont été décrits pour la première fois par B.J.J. Young et al. dans Br. Med. J. 2:97-99, (1979). Ces autoanticorps ont été à l'époque dénommés "anticorps antikératines". Jusqu'à présent, il était communément admis qu'ils étaient dirigés contre les cytokératines.

Ces autoanticorps spécifiques de la PR sont à l'heure actuelle détectés et titrés par immunofluorescence indirecte (IFI) sur cryocoupes transversales d'oesophage de rat.

Les techniques d'IFI présentent toutefois des inconvénients qui limitent leur mise en oeuvre pour le diagnostic de routine de la PR. En particulier, elles nécessitent l'utilisation de cryocoupes de tissu, ce qui implique une perte de temps et l'intervention de personnel spécialisé ; en particulier, l'analyse et l'interprétation fiable des résultats ne peuvent être faites que par des personnes familiarisées avec l'histologie.

Il est donc particulièrement souhaitable de disposer de préparations purifiées des antigènes reconnus par les autoanticorps spécifiques de la PR, utilisables dans des techniques classiques de diagnostic immunochimique.

G.Serre et al. dans Rev. Rhum. 53(11):607-614 (1986) ont montré qu'un antigène défini par ces auto-anticorps est caractéristique de certains épithéliums malpighiens cornifiés de mammifères. Présent principalement dans le *Stratum Corneum* de ces épithéliums, il est exprimé par exemple dans l'épiderme humain.

Toutefois, jusqu'à présent, on n'était parvenu ni à identifier précisément un tel antigène, ni à le purifier.

Les travaux effectués par les Inventeurs dans le domaine de la différenciation épidermique et malpighienne, notamment la production d'anticorps monoclonaux pour la définition de nouveaux antigènes de différenciation et l'étude des autoanticorps humains contre ces mêmes antigènes, leur ont permis de montrer que les anticorps anti-*Stratum Corneum* d'oesophage de rat sont d'authentiques autoanticorps, qui ne reconnaissent pas les cytokératines, mais d'autres antigènes exprimés en particulier dans l'épithélium oesophagien murin et l'épiderme humain.

Des travaux plus poussés ont maintenant permis aux Inventeurs d'extraire de l'épithélium oesophagien de rat et de l'épiderme humain, des antigènes portant des déterminants antigéniques spécifiquement reconnus par les autoanticorps présents chez les patients atteints de PR, de purifier et de caractériser biochimiquement ces antigènes.

Ces antigènes présentent les propriétés suivantes :
- ils sont susceptibles d'être extraits à partir de l'épiderme humain ou de l'épithélium oesophagien de rat,
- ils sont de nature protéique,
- un traitement par une nucléase ne modifie pas leur immunoréactivité,
- un traitement par l'éthanol ne modifie pas leur immunoréactivité,
- un traitement par l'acide trichloroacétique (TCA) ne modifie pas leur immunoréactivité,
- ils sont hydrosolubles,
- ils sont spécifiquement reconnus par les autoanticorps présents chez les patients atteints de PR.

Un premier antigène spécifiquement reconnu par les autoanticorps présents chez les malades atteints de PR a été extrait de l'épiderme humain.

Cet antigène est caractérisé plus particulièrement par les propriétés suivantes :
- après électrophorèse en gel de polyacrylamide (PAGE) en conditions natives, il se sépare en plusieurs protéines antigéniques qui migrent comme des témoins (qui sont des protéines globulaires) dont les poids moléculaires sont compris entre 80 et 400 kD environ, les protéines les plus immunoréactives se situant dans les zones comprises entre environ 80 et 120 kD ;
- après isoélectrofocalisation (IEF), il se dissocie en plusieurs protéines antigéniques dont les points isoélectriques (pHi) varient de 5,8 à 7,4.
- après électrophorèse bidimensionnelle du type IEF, suivie d'une migration en PAGE en présence de dodécyl sulfate de sodium (SDS), il apparaît comme une protéine antigénique de poids moléculaire apparent moyen d'environ 40 kD, dont le pHi varie de 5,8 à 7,4.

Les Inventeurs ont également constaté que cet antigène présente des propriétés immunologiques et biochimiques en commun avec la filaggrine humaine et correspond à un fragment de protéolyse de la profilaggrine (cette dernière étant le précurseur de la filaggrine).

A la suite de cette constatation, les Inventeurs ont entrepris de tester l'immunoréactivité de la filaggrine et de la profilaggrine humaine avec des auto-anticorps spécifiques de la PR, et ont mis en évidence une réactivité similaire à celle de l'antigène conforme à l'invention décrit ci-dessus.

La filaggrine est une famille de protéines qui a été identifiée chez diverses espèces au niveau des épithéliums kératinisants. Ce sont des protéines très basiques, riches en histidine. Elles dérivent de la déphosphorylation et de la protéolyse d'un précurseur, la profilaggrine, et subissent ensuite une maturation au cours de laquelle les résidus arginine basiques sont convertis en résidus citrulline neutres. Ce phénomène de maturation est en partie responsable du manque d'homogénéité des préparations de filaggrine qui, pour une même poids moléculaire, se présentent sous forme de plusieurs variants isoélectriques.

Le précurseur de la filaggrine, la profilaggrine, est constitué essentiellement de domaines répétés de filaggrine ; il en résulte une étroite parenté immunologique de ces deux molécules [pour revue sur les filaggrines, cf. DALE et al. Cellular and Molecular Biology of Intermediates Filaments, pp. 393-412, GOLDMAN et STEIWERT eds. Plenum Press (1990)].

La profilaggrine et la filaggrine sont extraites de l'épiderme par action de tampons à force ionique élevée et en présence d'urée. Toutefois, la filaggrine, une fois purifiée, est partiellement soluble dans des tampons de faible force ionique, à pH neutre.

Aucune similitude entre la filaggrine humaine et un antigène reconnu par les auto-anticorps anti-*Stratum Corneum* de classe G, dits "antikératines" spécifiques de la PR n'avait été suggérée jusqu'à présent. La seule relation qui a pu été établie par quelques auteurs est l'observation d'une colocalisation entre la filaggrine ou la profilaggrine, et le facteur périnucléaire (qui est un marqueur immunochimique de la polyarthrite rhumatoide). Ces auteurs sont toutefois parvenus à la conclusion que le facteur périnucléaire est différent de la filaggrine [HOET et al. Clin. Exp. Immunol. 84. 59-65 (1991)].

Les inventeurs ont également extrait d'épithélium oesophagien de rat un second antigène spécifiquement reconnu par les autoanticorps présents chez les patients atteints de PR.

Cet antigène présente les propriétés suivantes :
- après électrophorèse en PAGE-SDS, il se sépare en les deux protéines antigéniques suivantes :
   . une première protéine de poids moléculaire moyen apparent d'environ 210 kD,
   . une deuxième protéine de poids moléculaire moyen apparent compris entre environ 90 et 130 kD,
- après électrophorèse en PAGE en conditions natives, il se sépare en les trois protéines antigéniques suivantes :
   . une première protéine qui migre comme la ferritine de rate de cheval (poids moléculaire : 440 kD)
   . une deuxième protéine qui migre comme la catalase de foie de boeuf (poids moléculaire : 232 kD),
   . une troisième protéine qui migre entre la lactico-déshydrogénase de coeur de boeuf, et la sérum-albumine bovine (poids moléculaires respectifs : 140 kD et 67 kD).
- après électrophorèse bidimensionnelle du type IEF, suivie d'une migration en PAGE-SDS, il se dissocie en trois protéines antigéniques :
   . une première protéine de poids moléculaire moyen apparent d'environ 210 kD dont les pHi varient d'environ 5,85 à 6,85,
   . une deuxième protéine de poids moléculaire moyen apparent compris entre environ 90 et 130 kD, dont les pHi varient d'environ 5,85 à 7,35,
   . une troisième protéine dont le poids moléculaire moyen apparent varie d'environ 120 à 67 kD pendant que son pHi varie d'environ 5 à 7,5.
- après chromatographie séquentielle de gel filtration et d'interaction hydrophobe puis analyse en PAGE-SDS, cet antigène se sépare en deux fractions protéiques antigéniques d'hydrophobicité relative différente:
   . la fraction la plus hydrophobe contient la protéine de poids moléculaire moyen apparent d'environ 210 kD et la protéine de poids moléculaire moyen apparent compris entre environ 90 et 130 kD,
   . la fraction la moins hydrophobe contient la protéine de poids moléculaire moyen apparent compris entre environ 120 et 67 kD.

Les inventeurs ont en outre fait les constatations suivantes :
- L'antigène de l'oesophage de rat présente des réactions croisées immunologiques avec la filaggrine (et, bien entendu, la profilaggrine) humaine et la filaggrine extraite de l'épiderme de rat, mais il apparaît que ces réactions croisées n'impliquent pas les mêmes épitopes. En effet, la filaggrine extraite de l'épiderme de rat n'est pas reconnue par les auto-anticorps spécifiques de la PR ; il en résulte que ce ne sont pas les épitopes reconnus par ces autoanticorps qui interviennent dans les réactions croisées entre l'antigène de l'épithélium oesophagien de rat et la filaggrine épidermique de rat. Il en va tout autrement dans le cas des réactions croisées entre l'antigène épithélial oesophagien de rat et la filaggrine humaine, qui impliquent au moins une partie des épitopes reconnus par les autoanticorps spécifiques de la PR. Il est à noter également que les épitopes concernés par ces réactions croisées et reconnus par les autoanticorps spécifiques de la PR ne représentent qu'une partie des épitopes de la filaggrine humaine. Par exemple, l'anticorps monoclonal AKH1 [DALE et al. J. Invest. Dermatol 88, 306-313 (1987)] qui reconnaît un épitope de la filaggrine humaine ne reconnait pas l'antigène de l'épithélium oesophagien du rat ; d'autre part, lorsque l'anticorps AKH1 est utilisé en immunofluorescence indirecte sur des cryocoupes d'épiderme humain, le marquage observé est différent de celui obtenu lorsque l'on utilise des auto-anticorps anti-Stratum Corneum spécifiques de la PR.
- L'antigène épidermique humain, quant à lui, présente avec la filaggrine et la profilaggrine humaine des réactions croisées où interviennent des épitopes reconnus par les autoanticorps spécifiques de la PR, et également d'autres épitopes tel que celui reconnu par l'anticorps monoclonal AKH1.

Toutes les fractions protéiques décrites ci-dessus, constitutives des antigènes de l'épiderme humain et de l'oesophage de rat, ont été purifiées et testées avec les autoanticorps présents chez les patients atteints de PR. Il apparaît que ces fractions protéiques présentent isolément des propriétés immunologiques équivalentes à celles des antigènes dont elles sont issues.

L'Invention englobe également des fractions des antigènes de l'épiderme humain et de l'oesophage de rat portant des épitopes reconnus spécifiquement par les autoanticorps spécifiques de la PR.

Au sens de la présente Invention, on entend par fraction, toute préparation antigénique reconnue par des autoanticorps anti-*Stratum Corneum* spécifiques de la PR, et obtenue à partir de l'un des antigènes mentionnés ci-dessus, par une technique de fractionnement appropriée (électrophorèse, chromatographie, électrofocalisation, etc...). Ceci englobe les fractions obtenues à partir de préparations de filaggrine et de profilaggrine humaine, ainsi que les fractions plus spécifiquement décrites ci-dessus, obtenues à partir des antigènes hydrosolubles d'épiderme humain et de cellules épithéliales d'oesophage de rat.

L'Invention concerne en conséquence également les trois protéines mises en évidence après électrophorèse bidimensionnelle du type IEF suivie d'une migration en PAGE-SDS, de l'antigène extrait de l'épithélium oesophagien de rat.

Ces trois protéines ont également été mises en évidence dans les fractions protéiques obtenues après chromatographie séquentielle de gel filtration et interaction hydrophobe de l'antigène extrait d'épithélium oesophagien de rat, puis électrophorèse en conditions dénaturantes.

La première de ces trois protéines présente un poids moléculaire moyen apparent d'environ 210 kD et des pHi variant d'environ 5,85 à 6,85 ; la deuxième présente un poids moléculaire moyen apparent compris entre 90 et 130 kD et des pHi variant d'environ 5,85 à 7,35 ; la troisième présente un poids moléculaire moyen apparent compris entre 67 et 120 kD et des pHi variant d'environ 5 à 7,5.

L'Invention concerne aussi la protéine mise en évidence après électrophorèse bidimensionnelle du type IEF suivie d'une migration en PAGE-SDS, de l'antigène extrait d'épiderme humain. Cette protéine a un poids moléculaire d'environ 40 kD et des pHi variant d'environ 5,8 à 7,4.

Les travaux des Inventeurs aboutissent donc à l'obtention d'une série de nouveaux antigènes solubles reconnus spécifiquement par des auto-anticorps de classe G spécifiques de la PR, et à la mise en évidence d'une réaction spécifique desdits auto-anticorps avec la filaggrine humaine.

Ces travaux trouvent une application directe dans le diagnostic de la PR. En conséquence, l'Invention concerne également :
- Des compositions antigéniques pour le diagnostic de la présence d'autoanticorps dans un échantillon biologique d'un patient atteint de PR, lesquelles compositions sont caractérisées en ce qu'elles contiennent au moins un antigène pris dans le groupe constitué par : l'antigène extrait des cellules épithéliales de l'oesophage de rat ; l'antigène extrait de l'épiderme humain ; leurs fractions ou leurs fragments peptidiques, tels que définis ci-dessus;
- l'utilisation de la filaggrine, de la profilaggrine, de leurs fractions, ou de leurs fragments peptidiques, pour le diagnostic *in vitro* de la PR, en particulier pour la préparation de compositions antigéniques pour le diagnostic de la présence d'autoanticorps dans un échantillon biologique d'un patient atteint de PR.

Ces compositions permettent la formation d'un complexe antigène-anticorps entre les antigènes de l'Invention (protéines, fractions protéiques et fragments peptidiques) et les autoanticorps présents dans un échantillon biologique, tel que le sérum d'un sujet atteint de PR.

La présente Invention a pour objet un procédé de détection des auto-anticorps de classe G spécifiques de la PR dans un fluide biologique, lequel procédé est caractérisé en ce qu'il comprend au moins une étape au cours de laquelle on met en contact ledit fluide biologique avec au moins un antigène pris dans le groupe constitué par :
- la filaggrine humaine, la profilaggrine humaine ;
- un antigène extrait des cellules épithéliales de l'oesophage de rat, tel que défini ci-dessus ;
- un antigène extrait de l'épiderme humain, tel que défini ci-dessus ;
- les fractions ou les fragments peptidiques issus des antigènes ci-dessus ;
laquelle mise en contact est effectuée dans des conditions permettant la formation d'un complexe antigène-anticorps avec les auto-anticorps spécifiques de la PR éventuellement présents.

L'antigène est éventuellement marqué ou conjugué à une molécule porteuse.

A l'issue de cette étape, on procède à la détection dans le fluide biologique du complexe immunologique formé, par des méthodes physiques ou chimiques connues en elles-mêmes.

Le procédé de diagnostic précédent peut être mis en oeuvre grâce à un nécessaire ou kit comprenant :
- au moins un antigène (protéines, fractions et fragments) selon l'Invention, éventuellement marqué, ou un conjugué de cet antigène ou protéine avec une molécule porteuse ;
- des réactifs pour la constitution d'un milieu propice à la réaction immunologique avec les autoanticorps éventuellement présents dans un échantillon biologique ;
- un ou plusieurs réactifs pour la détection du complexe immunologique formé ;
- le cas échéant, des échantillons de référence, tels qu'un ou plusieurs sérum(s) négatif(s) et un ou plusieurs sérum(s) positif(s).

Selon un mode de mise en oeuvre préféré du procédé conforme à l'Invention, un test par immunotransfert pour la détection des autoanticorps anti-*Stratum Corneum* de classe G, spécifiques de la PR a été mis au point.

Un protocole d'immunotransfert, réalisé à partir d'un extrait antigénique épithélial oesophagien murin, a été optimisé pour obtenir une meilleure définition des bandes immunoréactives et une meilleure sensibilité, puis trois configurations différentes, soit par la méthode de séparation des protéines antigéniques (électrophorèse en conditions natives ou dénaturantes), soit par la dilution des sérums analysés, ont été validées sur le plan diagnostique avec une large série de sérums représentatifs de la pathologie rhumatologique.

Il est apparu que dans ces trois conditions, la sensibilité diagnostique du test oscillait autour de 50% pour une spécificité voisine de 95%, lorsque seule la présence ou l'absence d'immunoréactivité était prise en compte.

Par contre, la protéine antigénique migrant entre deux témoins de masses molaires 140 kD et 67 kD après séparation de l'antigène en conditions natives, reconnue beaucoup plus spécifiquement par les sérums de patients atteints de PR, a permis la détection de ceux-ci avec une sensibilité de 68% pour une spécificité de 99%, performance très supérieure à celle de l'immunofluorescence indirecte, qui présente une sensibilité de 43% pour une spécificité de 99%.

Une approche parallèle conduite avec l'antigène extrait d'épiderme humain a permis de montrer que la protéine de 40 kD, seule forme immunoréactive dans ce tissu, permettait d'obtenir des performances diagnostiques équivalentes à celles de la protéine antigénique murine migrant entre deux témoins de masses molaires 140 kD et 67 kD.

L'Invention est aussi relative à un procédé de purification des autoanticorps spécifiquement présents chez les patients atteints de PR, caractérisé en ce qu'il comprend au moins les étapes suivantes :
- la mise en contact d'un fluide biologique d'un patient atteint de PR avec au moins un antigène conforme à l'Invention, et/ou avec la filaggrine ou la profilaggrine humaine, ou avec leurs fractions ou leur fragments peptidiques, tels que définis dans ce qui précède, dans des conditions permettant la formation de complexes immunologiques avec lesdits anticorps ;
- l'isolement desdits complexes du milieu réactionnel ;
- la séparation des autoanticorps des complexes immunologiques.

L'Invention a également trait à un procédé de préparation d'anticorps polyclonaux caractérisé en ce qu'on utilise comme immunogène, au moins un des antigènes (protéines, fractions protéiques ou fragments peptidiques) de l'Invention. fractions protéiques ou fragments peptidiques) de l'Invention. Ces anticorps peuvent correspondre à des anticorps polyclonaux purifiés, ou à des anticorps monoclonaux, produits par tout hybridome

L'Invention englobe également un procédé de préparation d'anticorps caractérisé en ce qu'on utilise comme antigène d'immunisation la filaggrine ou la profilaggrine humaine, et en ce qu'on recueille les anticorps reconnaissant également l'antigène de l'épithélium oesophagien de rat. Ces anticorps peuvent correspondre à des anticorps polyclonaux purifiés, ou à des anticorps monoclonaux, produits par tout hybridome préparé selon les méthodes classiques de fusion cellulaire entre des cellules spléniques activées *in vitro* par l'antigène ou provenant d'un animal immunisé contre l'une ces protéines antigéniques de l'Invention et des cellules d'une lignée myélomateuse.

Il va de soi que l'Invention concerne également tous les anticorps obtenus par les procédés ci-dessus.

L'invention concerne aussi un procédé de préparation d'anticorps anti-idiotype, lequel procédé est caractérisé en ce qu'il comprend une étape au cours de laquelle on immunise un animal avec un anticorps monoclonal ou polyclonal obtenu conformément à l'Invention.

Des anticorps obtenus conformément à l'invention sont utilisables en particulier dans un but de recherche, pour étudier les mécanismes immunologiques associés à la PR, et dans la mise au point de traitements.

Ils peuvent être également utilisés pour localiser et caractériser des antigènes présentant des caractéristiques similaires à celles des antigènes de l'invention; en particulier des antigènes éventuellement présents dans des épithéliums malpighiens et dans les tissus articulaires de mammifères, y compris des mammifères d'autres espèces que l'homme ou le rat.

D'autres caractéristiques et avantages de l'Invention apparaîtront à la lecture des exemples qui suivent et qui sont illustrés par les figures en annexe étant entendu que ces exemples ne sauraient être interprétés comme tendant à réduire la portée des Revendications.

### EXEMPLE 1 - CARACTERISATION DE L'ANTIGENE EXTRAIT D'EPITHELIUM OESOPHAGIEN DE RAT

### I - MATERIEL ET METHODE

### A - EXTRACTION DES PROTEINES EPITHELIALES

### 1) Préparation de l'épithélium oesophagien par clivage chorio-épithélial

L'oesophage de rat est composé de trois tuniques principales qui sont, de l'extérieur vers l'intérieur : une couche conjonctive (l'adventice), une couche musculaire (la musculeuse), une couche composée d'un chorion recouvert par un épithélium malpighien cornifié (la muqueuse).

Après élimination, par dissection, de l'adventice et de la musculeuse, l'épithélium est séparé mécaniquement du chorion (clivage chorio-épithélial) après un choc thermique successivement à 4°C puis à 57°C en milieu liquide (KH₂/K₂H-PO₄ 8,5 mM; NaCl 150 mM; acide éthylène diamine tétraacétique (EDTA) 5 mM ; phénylméthyl sulfonylfluoride(PMSF) 4 mM; pH 7,4). Les épithéliums clivés sont conservés hydratés par un volume minimal de PBS (KH₂/K₂H-PO₄ 8,5 mM; NaCl 150 mM; pH 7,4) avant de subir un contrôle histologique permettant de vérifier l'absence de chorion contaminant.

### 2) Extraction des protéines épithéliales

### a) Tampons et séquences d'extraction

L'antigène de l'Invention étant exclusivement épithélial, c'est à partir du seul épithélium que sont réalisées les extractions.

A partir d'épithéliums clivés, l'extraction des protéines épithéliales est réalisée successivement par broyage au potter puis agitation à 4°Celsius.

Des protocoles classiques (W.W. Franke et al., J. Mol. Biol., 153:933-959, 1981 - T.T Sun and H. Green, J. Biol. Chem. 253(6):2053-2060, 1978) adaptés à l'extraction séquentielle des protéines à partir de tissus épithéliaux et notamment de l'épiderme, ont été tout d'abord utilisés. Puis des extractions en une seule étape ont ensuite été testées. Différents milieux d'extraction correspondant à des tampons Tris-HCl additionnés ou non de sels (KCl, NaCl), de détergents (Triton, Nonidet P40 [NP40]), d'agents dénaturants (urée, SDS) et d'agents réducteurs (bêta-mercaptoéthanol [2-ME]) ont ainsi été testés.

Au cours des extractions fractionnées, l'antigène a été retrouvé exclusivement dans la fraction correspondant soit à un tampon Tris de faible force ionique, soit à un tampon Tris salin (TBS) additionné d'EDTA et de Triton. Ceci a permis de confirmer que l'antigène n'est pas une cytokératine, de montrer qu'il est extractible en absence d'urée et d'agents réducteurs et qu'il est hydrosoluble.

Cependant, en comparant les diverses techniques d'extraction, l'intensité de l'immunoréactivité est apparue supérieure en présence de NP4O. Un milieu d'extraction constitué de TBS + inhibiteurs + NP4O à 0,5 % apparaît donc comme le plus efficace.

Lors de la purification de l'antigène, l'intense absorption du NP4O à 280 nm interfère avec celle des protéines, notamment en chromatographie de gel filtration. Un tampon TBS + inhibiteur sans NP4O peut être utilisé pour l'extraction de l'antigène en préalable à des travaux de purification.

### b) Optimisation des conditions physiques d'extraction

Afin d'optimiser la procédure d'extraction, certains paramètres physiques ont été explorés de manière systématique. Il s'agit de la température, de la durée, du type de broyage, du type d'agitation et de l'action d'inhibiteurs enzymatiques.

### - Broyage :

Il a été effectué soit au potter électrique à haute vitesse, soit de façon plus douce, au potter manuel. L'efficacité de l'extraction a été dans les deux cas à peu près équivalente. Le potter électrique est cependant préféré car il permet le broyage d'un plus grand nombre d'oesophages.

### - Sonication :

L'optimisation de l'extraction par des sonications de durée variable, après potterisation a été testée. Elle s'est avérée très faible. La sonication n'est donc pas nécessaire.

### - Agitation :

Agitation douce et agitation rapide ont été comparées. Une agitation rapide en présence d'un détergent entraînant la formation de mousse provoque une suroxygénation du milieu susceptible d'altérer l'antigène. En fait, la qualité de l'extraction a été identique dans les deux cas. Une agitation douce a cependant été retenue pour préserver au maximum l'antigène.

### - Température :

Température ambiante et température de 4°Celsius ont été comparées. De meilleurs résultats ont été obtenus avec des extractions conduites à 4°Celsius.

### - Durée :

Des extractions sous agitation de 15 minutes, 2 heures et 17 heures ont été comparées. Bien que l'augmentation du temps d'extraction augmente faiblement la quantité d'antigène extrait, une durée de 15 minutes a été retenue pour minimiser le risque d'altération de l'antigène.

### - Inhibiteurs :

L'effet protecteur pour l'antigène des inhibiteurs suivants a été testé :
- Inhibiteur de la prolifération bactérienne : Azide de sodium,
- Inhibiteur d'enzymes lytiques :
   protéases (aprotinine, PMSF), phosphatases (NaF, PCMB (acide parachloromercuribenzoïque)), glycosidases (déoxymannojirimycine, déoxyjirimycine).

Des extractions sans inhibiteurs ont été réalisées notamment lorsque l'extrait était destiné à la caractérisation biochimique de l'antigène par action de différentes enzymes ; aucune modification de l'antigène n'a été constatée en l'absence d'inhibiteur.

### - Protocole d'extraction optimisé :

L'étude des paramètres précédents a permis de déterminer le protocole d'extraction optimisé ci-après :
- Substrat : épithélium d'oesophage de rat obtenu par clivage chorio-épithélial.
- Tampon : Tris-HCl 40 mM
   NaCl 150 mM
   EDTA 10 mM
   pH 7,4
- Inhibiteurs : Azide de sodium 0,1 %
   PMSF 1 mM
   Aprotinine 2 µg/ml
- Rapport substrat/tampon :
   500 µl de tampon par épithélium oesophagien
- Conditions :
   Broyage : potter électrique, 15 minutes
   Température de broyage : 4°Celsius
   Agitation après broyage : 15 minutes à 4°Celsius
- Concentration protéique totale de l'extrait :
   5 à 8 mg/ml
- Conservation de l'extrait brut :
   - 20 ou -40° Celsius

### - Préparation à partir de muqueuse oesophagienne totale :

Dans un but de simplification de la procédure d'extraction, l'extraction de l'antigène à partir de muqueuse oesophagienne totale a été étudiée. Une simple dissection de la muqueuse sans clivage chorio-épithélial a été réalisée. La contamination protéique de l'extrait ainsi induite a été étudiée.

Des extractions parallèles, soit à partir de muqueuses, soit à partir d'épithéliums clivés ont été effectuées.

L' analyse en PAGE-SDS des protéines extraites montre que la présence du chorion n'entraîne pas de contamination protéique majeure dans la zone de masse molaire de l'antigène. Après immunotransfert de la fraction épithélio-choriale extraite par broyage, l'antigène est parfaitement détectable.

### C) Concentration de l'extrait brut

L'antigène étant faiblement représenté dans l'extrait brut (moins de 1 % des protéines totales), une étape de concentration des extraits s'avère nécessaire, surtout lorsque ceux-ci doivent être analysés par électrophorèse miniaturisée de type système "Phastsystem Pharmacia^{(R)}" (dépôts de l'ordre du microlitre).

Parmi les diverses techniques de concentration testées, celle consistant à concentrer les extraits par précipitation au TCA 10 % a été retenue. Cette technique facile à mettre en oeuvre ne produit aucune altération de l'antigène puisque la réponse immunologique est parfaitement conservée en immunotransfert.

De la même façon, des précipitations par l'éthanol ou le sulfate d'ammonium ainsi que la lyophilisation contrôlée n'induisent aucune altération sensible de l'antigène et sont donc parfaitement utilisables.

### B - ELECTROPHORESES

Quels que soient les milieux d'extraction étudiés (comme décrit ci-avant), les protéines solubles sont séparées par PAGE puis électro-transférées sur nitrocellulose.

### 1) Séparation par IEF horizontale

. Gel : 9 X 15 cm, 4% acrylamide contenant des ampholines formant un gradient de pH de 3,5 à 9,5.
. Echantillon déposé : extrait brut préparé selon le protocole précédent, concentré par précipitation au TCA à 10% et repris dans l'eau.

### 2) Electrophorèses monodimensionnelles

- Electrophorèse verticale classique selon la méthode décrite par Laemmli (Nature, 227 : 680-685, 1970)
   . Gel : 10 X 10 cm, 7% acrylamide.
   . Tampon de migration : Tris 25 mM, glycine 192 mM, SDS 1%, pH 8,3.
   . Echantillon déposé : extrait brut lyophilisé et repris en Tris-HCl 10 mM pH 7,4, SDS 2%, 2ME 1%, bleu de bromophénol 0,1%, glycérol 10%.
- Electrophorèse horizontale miniaturisée en conditions dénaturantes.
   . Gel : 4 X 4 cm, 7,5% acrylamide.
   . Tampon Tris 200 mM, Tricine 200 mM, SDS 0,55%, pH 8,1.
   . Echantillon déposé : extrait brut concentré par précipitation au TCA 10% et repris en Tris-HCl 10 mM, pH 7,4, SDS 2%, 2-ME 1%, Bleu de bromophénol 0,1%.
- Electrophorèse horizontale miniaturisée en conditions natives.
   . Gel : 4 X 4 cm, gradient de 8 à 25% en acrylamide.
   . Tampon de migration : Tris 250 mM, L-Alanine 880 mM, pH 8,8.
   . Echantillon déposé : extrait brut concentré par précipitation au TCA 10% et repris soit dans l'eau, soit en tampon Tris-HCl 200 mM à différents pH.

### 3) Electrophorèses bidimensionnelles IEF/PAGE-SDS

- Première dimension : IEF horizontale.
   . Gel : 4 X 4 cm, 4% acrylamide, gradient de pH 3,5 à 9,5.
   . Echantillon déposé : extrait brut concentré par précipitation à l'éthanol (4 volumes) et repris dans l'eau.
- Deuxième dimension : électrophorèse dénaturante comme décrite précédemment.
   . Echantillon déposé : piste gel IEF incubée en Tris 112 mM, acide acétique 112 mM, SDS 2,5%, 2-ME 1%.

### C - IMMUNODETECTION

L'immunodétection de l'antigène est réalisée à l'aide de sérums humains, provenant de patients atteints de PR, dans lesquels les autoanticorps anti-*Stratum Corneum* d'oesophage de rat ont été préalablement titrés par IFI semiquantitative et affectés d'une valeur-titre de 0 à 8 : paramètre GIFOR (G-Immunofluorescence-Oesophage de Rat). La valeur-seuil, GIFOR = 2, qui correspond à une spécificité diagnostique pour la PR supérieure à 99 % permet de classer les sérums de malades en deux groupes : GIFOR+ et GIFOR- (C.Vincent et al., Ann. Rheum. Dis. 48:712-722, 1989).

Dans les sérums choisis, on a également pris en compte le paramètre immunologique suivant : le titre des autoanticorps anticytokératines épidermiques de classe G déterminé en ELISA (GELISA) (G.Serre et al., J. Invest. Dermatol. 88:21-27, 1987). Ces autoanticorps réagissent en immunotransfert avec les cytokératines humaines contaminantes éventuellement présentes dans l'extrait et dues à la desquamation naturelle des manipulateurs. Les sérums utilisés ont été choisis pour constituer également deux groupes, GELISA+ et GELISA-, en fonction du titre élevé (absorbance>0,6) ou faible (absorbance<0,2) de ces autoanticorps naturels. Au total, les quatre sous-groupes de sérums de PR suivants ont été utilisés :
- GIFOR+/GELISA+
- GIFOR+/GELISA-
- GIFOR-/GELISA+
- GIFOR-/GELISA-

Enfin, des sérums témoins provenant d'individus normaux ou de patients atteints d'affections rhumatologiques non rhumatoïdes ainsi que des anticorps monoclonaux murins spécifiques des cytokératines humaines ont été inclus dans les tests (G.Serre et al., Colloque INSERM, John Libbey Eurotext, Vol. 171:524, 1988).

Après PAGE des protéines, immunotransfert sur membrane de nitrocellulose et incubation avec les sérums humains ou les anticorps monoclonaux, un second anticorps, correspondant à des fragments F(ab)'2 de chèvre anti-IgG (gamma) humaines ou Fab de mouton anti-IgG (H+L) de souris, marqué à la peroxydase, est incubé avec la membrane et la formation du complexe antigène-anticorps est révelée par mise en évidence de l'activité peroxydasique, par H₂O₂ et 4-chloro-1-naphtol.

Dans ces conditions expérimentales, l'antigène est repérable en immunotransfert par une immunoréactivité spécifique des sérums de PR GIFOR+. Les autoanticorps naturels anticytokératines se traduisent, seulement lorsque l'antigène a été préalablement séparé en PAGE-SDS, par la présence de bandes immunoréactives situées autour de 65-67 Kd, présentes dans les seuls sérums GELISA+, plus ou moins intenses en fonction de leur titre et totalement indépendantes de la valeur de GIFOR.

### D - CHROMATOGRAPHIE

Les molécules antigéniques ont été séparées dans différentes fractions après chromatographie séquentielle gel filtration/interaction hydrophobe. Ces fractions ont été analysées par PAGE-SDS et PAGE en conditions natives.

### E - DIGESTION ENZYMATIQUE

### 1) Protéinase K

Deux extraits d'épithélium d'oesophage de rat en tampon TBS/NP40 sont précipités au TCA 10% et les protéines reprises dans un tampon Tris-HCl 50 mM pH 7,5 CaCl₂ 10 mM. Un des extraits est additionné de protéinase K (préincubée pendant 1 heure à 37°C pour éliminer d'autres hydrolases éventuellement présentes dans la préparation commerciale) à la concentration finale de 50 g/ml. Les deux échantillons sont ensuite incubés pendant 1 heure à 37°C puis analysés comparativement en immunotransfert après migration en PAGE en conditions natives ou en PAGE-SDS.

### 2) Nucléase

Deux extraits d'épithélium d'oesophage de rat en tampon TBS/NP40 sont précipités au TCA 10%. L'un d'eux est hydrolysé par 2 U/µl de nucléase de microccoque (hydrolyse de l'ADN et de l'ARN) en Tris-HCl 50 mM, pH 7,5, CaCl₂ 5 mM. Le deuxième échantillon, non traité, sert de témoin. Les deux préparations sont incubées pendant 1 heure à 37°C puis analysées comparativement en immunotransfert après migration en PAGE en conditions natives ou en PAGE-SDS.

### F - DEGLYCOSYLATION

### 1) Oxydation à l'acide périodique

Le protocole de WOODWARD et COLL. (J. IMM. METH. 78:143-153, 1985) a été utilisé ; ce protocole permet l'oxydation periodique d'un échantillon préalablement transféré sur une membrane de nitrocellulose, l'oxydation periodique pouvant être suivie d'une immunodétection.

Un extrait d'épithélium d'oesophage de rat est transféré après migration en PAGE-SDS. La membrane de nitrocellulose est découpée en plusieurs pistes qui sont soumises à une oxydation periodique (incubation en tampon acétate de sodium 50 mM pH 4,4, periodate de sodium 20 mM, pendant 1 heure à l'obscurité et à température ambiante) suivie d'une réduction au borohydrure de sodium (NaBH₄) 50 mM en PBS pH 7,4 pendant 30 minutes à température ambiante). Des échantillons témoins ne subissant aucun traitement chimique ou subissant uniquement la réduction ont été inclus. L'ensemble des échantillons est ensuite immunodétecté.

### 2) Traitement par l'acide trifluorométhanesulfonique (TFMS)

L'utilisation du TFMS, selon la méthode de EDGE et Coll. (ANAL. BIOCHEM. 118:131-137, 1981), permet une déglycosylation quasi-totale des glycoprotéines tout en conservant leur axe peptidique. Cette méthode a été appliquée à une glycoprotéine témoin, la fétuine bovine, et à des extraits d'épithélium d'oesophage de rat contenant l'antigène. Les extraits sont dialysés contre de l'eau puis lyophilisés. Leur traitement est réalisé par addition d'un mélange (66/33-V/V) de TFMS et d'anisole (agent protecteur de l'axe peptidique) et incubation sous agitation après saturation en azote de l'atmosphère des tubes. Le protocole de référence a été optimisé en faisant varier différents paramètres :
- proportion de mélange TFMS/anisole par rapport à la masse de protéines traitée : 33 à 266 µl de mélange par mg de protéine totale de l'extrait oesophagien.
- température : 0°C, 4°C ou température ambiante.
- Durée de l'incubation : 1, 3 ou 24 heures.

Dans tous les cas, après incubation, l'arrêt de la réaction est effectué par addition de 2 volumes d'éther glacial et de 3 volumes d'un mélange pyridine/eau (50/50-V/V). Le précipité de trifluorométhanesulfonate de pyridinium, soluble dans la phase éthérée, a été éliminé avec elle. Après une nouvelle extraction par l'éther glacial, la phase aqueuse a été dialysée contre de l'eau. Les échantillons ont ensuite été lyophylisés, séparés par PAGE, transférés, puis immunodétectés.

### 3) Traitement par la peptide N-glycosidase F (PNGase F)

La PNGase F, également commercialisée sous le nom de Glycopeptidase F ou de N-Glycanase, hydrolyse la liaison amide existant entre le résidu asparaginyl d'une glycoprotéine et le N,N'-diacétyl-chitobiose de la structure commune à tous les oligosaccharides N-liés.

La fétuine contient trois chaînes oligo-saccharidiques N-liées dont l'élimination par la PNGase F entraîne une diminution de masse molaire facilement visualisable en PAGE-SDS (S. HIRANI et al., ANAL. BIOCHEM. 162:485-492, 1987).

La fétuine bovine (2 mg/ml) et un extrait lyophilisé d'épithélium oesophagien de rat (8 mg/ml de protéine) sont dénaturés pendant 5 mn à 90°C avec du SDS à 2% et du 2-ME 0,05 M. Puis les protéines sont placées dans un milieu réactionnel de composition suivante :
- NP40 1,25% (protection de l'enzyme vis-à-vis de l'action dénaturante du SDS),
- phosphate de potassium 166 mM pH 8 (conservation du pH optimal de l'enzyme),
- 1,10-orthophénantroline 10 mM (inhibiteur de protéases) solubilisé dans du méthanol à la concentration finale de 10%,
- PNGase F 0,3 U/µl.

Dans ce milieu réactionnel, les concentrations finales respectives du SDS et du 2-ME sont de 0,166 % et 16 mM. L'incubation est de 24 heures à 37°C.

Des témoins "fétuine" et "extrait antigénique" incubés sans enzyme sont inclus dans le protocole. Après hydrolyse et concentration par précipitation au TCA, les échantillons sont séparés par PAGE, transférés et immunodétectés.

### G - REACTIVITE AVEC DIFFERENTES LECTINES

Les Inventeurs ont cherché à confirmer la nature glycoprotéique de l'antigène en détectant spécifiquement certains sucres par des lectines couplées à la peroxydase. Deux lectines végétales : la lectine de germe de blé (WGA) et la concanavaline A (ConA) ont été choisies pour leur spécificité différente (I.J. GOLSTEIN et R.D. PORETZ, The Lectins : properties, functions and applications in biology and medicine)
- la WGA reconnaît des résidus de (D-N acétylglucosamine avec les affinités croissantes suivantes :
   (1) GlcNAc<<GlcNAcβ1-4GlcNAc<GlcNAcβ1-4 GlcNAcβ1-4GlcNAc et se fixe également avec une moindre affinité sur les résidus d'acide sialique.
- La Con A s'associe aux sucres suivants, classés par affinité croissante :
   (2) αGlcNAc<αGlc<αMan<Manα1-2Man<Manα1-2Manα1-2Man
les protéines de deux extraits antigéniques, bruts ou purifiés, sont transférées sur nitrocellulose après migration en PAGE monodimensionnelle ou IEF/PAGE-SDS. Les bandes de nitrocellulose ont été incubées avec la Con A et la WGA couplées à la peroxydase aux concentrations minimales respectives de 20 et 15 g/ml (J.C.S. CLEGG, ANAL. BIOCHEM 127:389-394, 1982 et W.F. GLASS et al., ANAL. BIOCHEM. 115:219-224, 1981). L'incubation est réalisée dans un tampon Tris-HCl 50 mM pH 7,4, NaCl 200 mM, CaCl₂ 1mM, MnCl₂ 1 mM et Tween 0,1% pendant 1 heure à 37°C et 1 nuit à 4°C. Celle-ci est précédée et suivie d'étapes de lavages dans le même tampon. Différents agents de saturation supplémentaires comme la gélatine ou la sérum-albumine bovine oxydée par un traitement à l'acide periodique ne permettent pas d'amplifier la réponse obtenue lors de l'utilisation du seul Tween. Après le dernier lavage, l'association lectine-antigène est révélée par mise en évidence de l'activité peroxydasique par H₂O₂ et 4-chloro-1-naphtol comme lors des immunodétections.

### II - CARACTERISATION PHYSICOCHIMIQUE DE L'ANTIGENE

La caractérisation physico-chimique de l'antigène a été effectuée à partir d'extraits bruts d'épithélium d'oesophage de rat, puis à partir de fractions antigéniques purifiées. Dans les deux cas, après séparation électrophorétique, les protéines ont été transférées puis immunodétectées par des sérums de patients atteints de PR.

### A - MASSE MOLAIRE

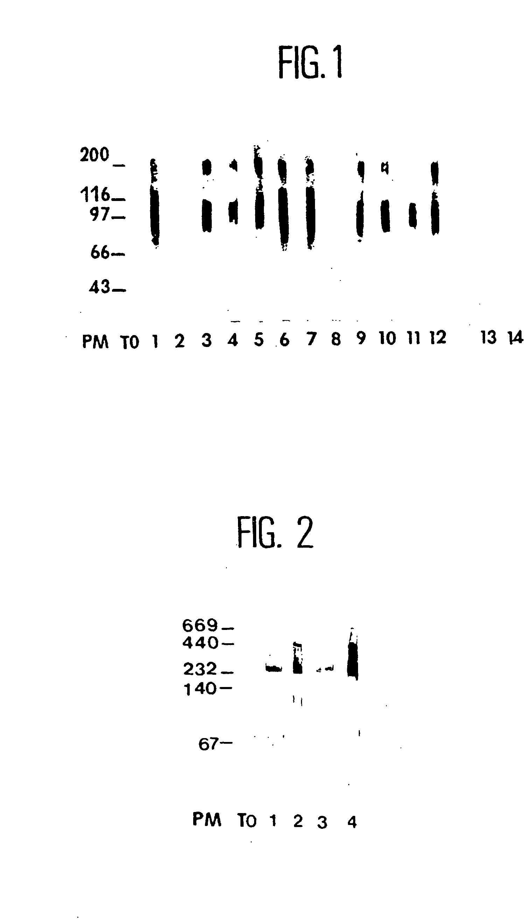

La figure 1 représente l'analyse en immunotransfert d'un extrait d'épithélium d'oesophage de rat séparé par PAGE-SDS. L'extrait brut est concentré dix fois par précipitation au TCA à 10%, repris en tampon de Laemmli, séparé par PAGE-SDS (7,5%), transféré sur nitrocellulose et immmunodétecté avec des sérums de PR GIFOR + (pistes 1-12) et GIFOR - (pistes 13,14) dilués au 1/100 ; "T₀" représente un témoin négatif incubé avec l'anticorps secondaire seul et "PM" représente des marqueurs de poids moléculaire. L'antigène se présente sous la forme d'une bande immunoréactive étroite correspondant à une protéine de masse molaire moyenne d'environ 210 kD et d'une zone immunoréactive large et étalée correspondant à une protéine de masse molaire moyenne comprise entre 90 et 130 kD.

### B - POINTS ISOELECTRIQUES

L'extrait brut est concentré cinq fois par précipitation au TCA à 10%, repris dans l'eau, séparé par IEF (pHi 3,5 à 9,5), transféré puis immunodétecté avec des sérums de patients atteints de PR, GIFOR + et GIFOR -. Les molécules antigéniques présentent une distribution très hétérogène, les protéines de l'extrait brut reconnues spécifiquement par la plupart des sérums GIFOR + se répartissent en un faisceau de bandes fines dont les points isoélectriques varient de 5,2 à 7,5.

### C - CARACTERISTIQUE DE MIGRATION EN CONDITIONS NATIVES

La figure 2 représente l'immunotransfert d'un extrait d'épithélium d'oesophage de rat repris en milieu acide et séparé par PAGE en conditions natives. L'extrait brut concentré dix fois par précipitation au TCA à 10%, est repris dans l'eau (pH inférieur à 4), séparé par PAGE en conditions natives (8-25%), transféré puis immunodétecté avec des sérums de PR GIFOR + (pistes 1-4) dilués au 1/100 ; "T₀" représente un témoin négatif incubé avec l'anticorps secondaire seul. Dans ces conditions de reprise de l'échantillon à pH acide, l'immunotransfert effectué avec quatre sérums de patients atteints de PR fait apparaître trois zones immunoréactives correspondant respectivement à :
. une première protéine qui migre comme la ferritine de rate de cheval (poids moléculaire : 440 kD)
. une deuxième protéine qui migre comme la catalase de foie de boeuf (poids moléculaire : 232 kD),
. une troisième protéine qui migre entre la lactico-déshydrogénase de coeur de boeuf, et la sérum-albumine bovine(poids moléculaires respectifs: 140 kD et 67 kD,
lesquelles sont reconnues avec une affinité variable selon les sérums.

### D - ELECTROPHORESE BIDIMENSIONNELLE IEF/PAGE-SDS

Cette technique qui combine IEF dans la première dimension et PAGE-SDS dans la deuxième permet de déterminer simultanément les points isoélectriques et les masses molaires des diverses molécules antigéniques, de confirmer leur grande hétérogénéité de charge et de montrer leur grande hétérogénéité de masse.

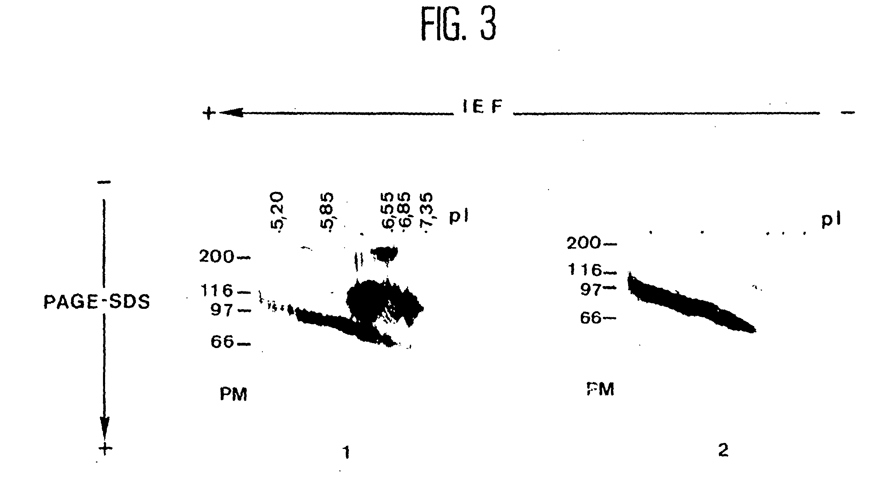

La figure 3 représente l'immunotransfert d'un extrait d'épithélium d'oesophage de rat séparé par électrophorèse bidimensionnelle (IEF/PAGE-SDS). L'extrait brut concentré 20 fois par précipitation à l'éthanol puis repris dans l'eau, est séparé par IEF (pHi 5 à 8) puis par PAGE-SDS (7,5%), transféré puis immunodétecté avec deux sérums de patients atteints de PR GIFOR + (1,2) dilués au 1/100. La zone immunoréactive de 90 à 130 kD présente des pHi variant de 5,85 à 7,35, ceux de la bande de 210 kD varient de 5,85 à 6,85. Une troisième population immunoréactive, en forme de "virgule" migre de 120 à 67 kD tandis que ces pHi se répartissent de manière continue entre 5 et 7,5. Les molécules antigéniques de masse molaire inférieures à 90 kD, clairement détectées après électrophorèse bidimensionnelle, n'apparaissent en électrophorèse monodimensionnelle qu'avec des sérums de titre élevé, sous la forme d'une extension de l'immunoréactivité vers les zones de faible poids moléculaire. La plus grande quantité d'antigène déposée en IEF/PAGE-SDS, permettant une meilleure sensibilité d'immunodétection, explique cette apparente discordance.

### E - CARACTERISTIQUES DE SEPARATION CHROMATOGRAPHIQUE

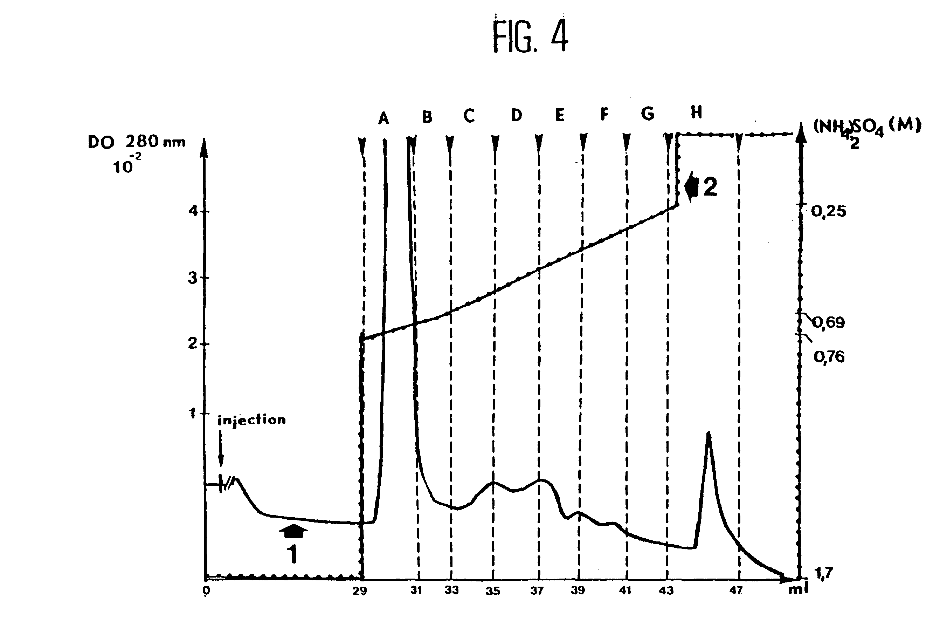

La figure 4 représente la séparation en chromatographie d'interaction hydrophobe d'une solution antigénique prépurifiée d'un extrait brut par chromatographie de gel filtration sur superose 12 (profil d'élution suivi par mesure de l'absorbance à 280 nm). Des fractions immunoréactives ainsi prépurifiées sont séparées sur Phényl-Superose (Tracé 2 = Tampon d'équilibre : phosphate 50 mM, (NH₄)₂SO₄ 1,7 M, pH 7 ; Tampon d'élution : phosphate 50 mM, pH 7 ; volume injecté : 3,6 ml ; Débit : 0,5 ml/mn ; DO 280 nm pleine échelle (Tracé 1) : 0,05.

Les profils d'élution et l'étude par immunotransfert des différentes fractions antigéniques se sont avérés très reproductibles au cours de multiples purifications. La répartition des molécules antigéniques dans les différentes fractions a donc pu être modelisée.

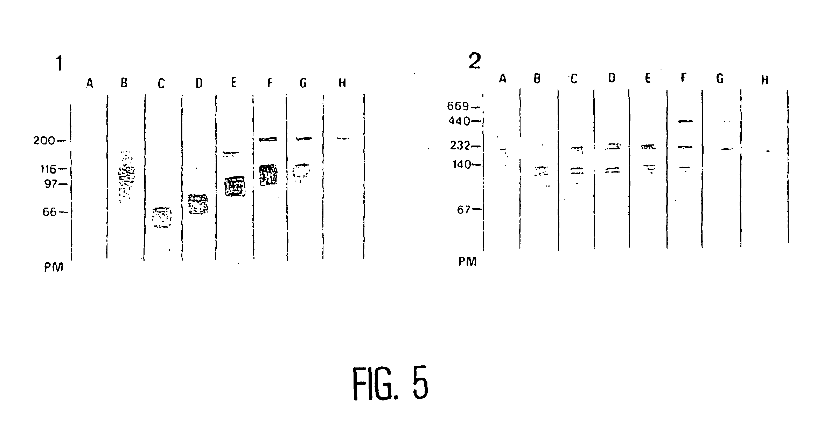

La figure 5 représente la modélisation des profils d'immunoréactivité des fractions antigéniques purifiées par chromatographie séquentielle gel filtration/interaction hydrophobe, obtenue après PAGE-SDS (1) ou PAGE en conditions natives (2). Les différences d'intensité des bandes ou zones immunoréactives reflètent la richesse relative en protéines antigéniques de chaque fraction (A-H).

En électrophorèse dénaturante, la bande à 210 kD (fractions F, G et H), la large bande mal limitée de 90 à 130 kD (fractions D, E, F, G et H) et une bande de 67 à 90 kD (fractions C, D et E) ont été visualisées.

En électrophorèse en conditions natives, la bande fine à 440 kD (fractions F, G et H), la bande à 232 kD (fractions C, D, E, F, G et H) et la large bande entre les témoins de 140 kD et 67 kD (fractions B, C, D et E) ont été retrouvées.

La comparaison des molécules antigéniques analysées en PAGE-SDS dans les différentes fractions purifiées (Figure 5) et des molécules identifiées après électrophorèse bidimensionnelle (IEF/PAGE-SDS) dans un extrait brut, comme indiqué à la figure 3, renseigne sur leur hydrophobicité respective :
- La bande à 210 kD dont les pHi varient de 5,85 à 6,85, est retrouvée dans les fractions obtenues en fin d'élution (fractions F, G et H), elle est donc très hydrophobe.
- La zone diffuse de 90 à 130 kd, dont les pHi se situent de 5,85 à 7,35, présente le même profil d'élution que la bande à 210 kD (fractions E, F, G et H) et donc la même hydrophobicité.
- Par contre, la population en forme de "virgule" qui présente des pHi plus étalés de 5 à 7,5, est retrouvée majoritairement en début d'élution (fractions B, C, D et E) et présente donc un caractère moins hydrophobe que les deux molécules précédentes.

### III - CARACTERISATION BIOCHIMIQUE DE L'ANTIGENE

### A - LES MOLECULES ANTIGENIQUES SONT DE NATURE PROTEIQUE

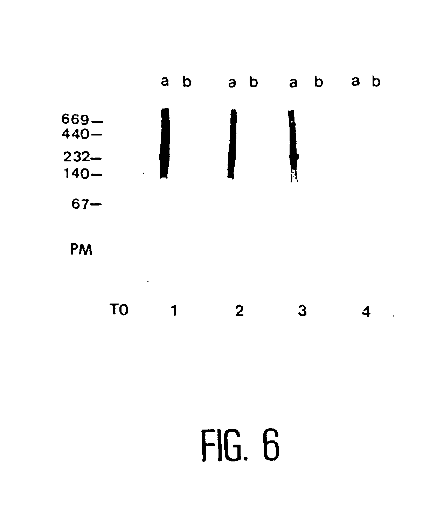

La figure 6 représente l'analyse en immunotransfert des protéines d'un extrait antigénique digéré (b) ou non (a) par la protéinase K, séparé par PAGE en conditions natives (8-25%), transféré puis immunodétecté avec trois sérums de patients atteints de PR GIFOR + (pistes 1-3) et un sérum de patient atteint de PR GIFOR - (piste 4) ; T₀ représente un témoin négatif incubé avec l'anticorps secondaire seul.

Les colorations au Rouge Ponceau des bandes de nitrocellulose correspondant aux échantillons traités montrent la digestion totale des protéines de l'extrait. L'immunodétection après PAGE en conditions natives montre que l'immunoréactivité spécifique des sérums GIFOR + disparaît lorsque l'échantillon est préalablement digéré par la protéinase K. L'immunodétection après transfert des échantillons séparés en PAGE-SDS a confirmé ce résultat.

La nature protéique des molécules porteuses de l'antigénicité étant affirmée, les Inventeurs ont entrepris de déterminer s'il s'agissait d'homo- ou d'hétéroprotéines, quelles associations et/ou substitutions de l'antigène étaient susceptibles de rendre compte de la diversité de point isoélectrique et de masse molaire apparente et quelle était la nature biochimique du ou des épitopes reconnus.

### B - LES PROTEINES ANTIGENIQUES NE SONT PAS ASSOCIEES A DES ACIDES NUCLEIQUES

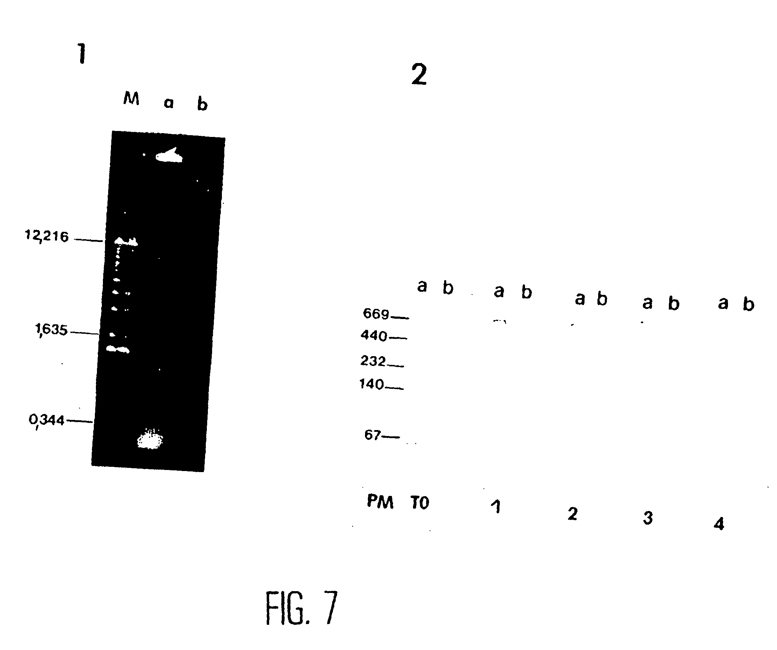

L'association éventuelle de l'antigène avec des acides nucléiques a été étudiée. La figure 7 représente en (1) l'analyse d'un extrait d'épithélium d'oesophage de rat traité (b) ou non (a) par la nucléase de microccoque puis analysé par électrophorèse en gel d'agarose 1% suivie d'une coloration au bromure d'éthidium, "M" représente un marqueur de taille en kilo-paires de bases.

Après séparation des échantillons par électrophorèse en gel d'agarose 1%, l'absence d'acides nucléiques dans l'échantillon traité prouve l'efficacité de la digestion enzymatique.

La figure 7 représente en (2) l'analyse par immunotransfert après PAGE en conditions natives (8-25%), réalisée avec trois sérums de patients atteints de PR GIFOR + (pistes 1-3) et un sérum de PR GIFOR - (piste 4) ; T₀ représente un témoin négatif incubé avec l'anticorps secondaire seul.

L'immunodétection après PAGE en conditions natives montre l'insensibilité des protéines antigéniques au traitement par la nucléase tant du point de vue de leur immunoréactivité avec les sérums de patients de PR que de celui de leur masse molaire apparente, prouvant qu'elles ne sont pas associées à des acides nucléiques.

### C - LES PROTEINES ANTIGENIQUES SONT-ELLES DES GLYCOPROTEINES ?

Les glycoprotéines présentent souvent une forte hétérogénéité de points isoélectriques et un taux inconstant de fixation du SDS qui rend médiocre leur résolution en PAGE-SDS. Les protéines antigéniques présentant également ces caractéristiques, les Inventeurs ont cherché leur éventuelle substitution par des résidus glucidiques. Dans ce but, des techniques visant soit à altérer les molécules de sucres éventuellement portées par l'axe peptidique, soit à débarrasser la partie protéique de ses résidus glycaniques, ont été utilisées.

### 1) Destruction d'éventuels épitopes glucidiques par l'acide periodique

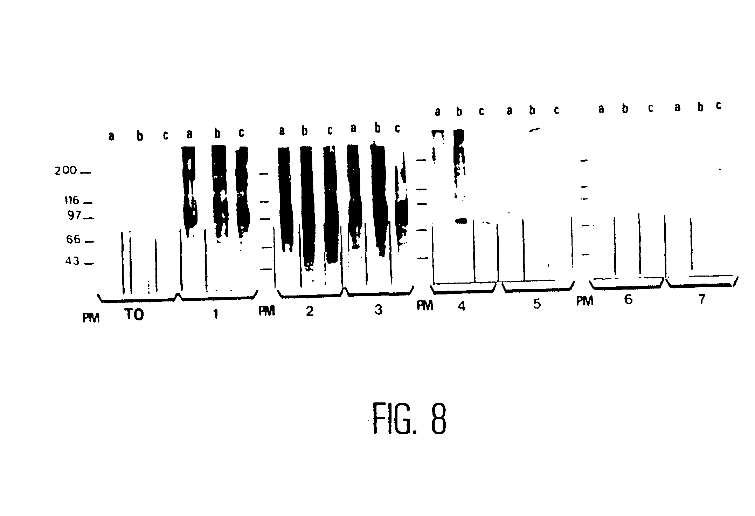

La figure 8 représente l'immunodétection d'un extrait d'épithélium d'oesophage de rat qui, après séparation par PAGE-SDS (7,5%) et transfert a été :
- incubé pendant 1h30 en tampon PBS (témoin "incubation" : piste a),
- incubé pendant 1h en tampon acétate de sodium, puis réduit pendant 30 minutes au borohydrure de sodium (témoin "réduction" : piste b),
- oxydé par l'acide periodique en tampon acétate de sodium pendant 1h puis réduit pendant 30 minutes au borohydrure de sodium ("essai" : piste c),
   trois sérums de PR GIFOR + (pistes 1-3), deux sérums de PR GIFOR - (pistes 4, 5) et deux sérums de contrôle (pistes 6, 7) ont été utilisés ; "T₀" représente un témoin négatif incubé avec l'anticorps secondaire seul.

Le traitement par l'acide periodique n'a en rien modifié les protéines spécifiquement reconnues par les sérums de patients atteints de PR. Trois interprétations de ce résultat sont possibles :
- les protéines antigéniques sont des glycoprotéines mais les sucres sensibles à une oxydation periodique qu'elles portent ne sont pas impliqués dans le ou les épitopes majoritairement reconnus par les autoanticorps spécifiques de la PR.
- les protéines antigéniques sont des glycoprotéines mais les sucres qu'elles portent de par leur nature et/ou leur type d'enchaînement sont insensibles à une oxydation periodique (l'acide periodique clive la liaison entre deux carbones vicinaux portant initialement soit chacun une fonction alcool, soit l'un une fonction alcool et l'autre une fonction acide ou cétone ou amine primaire ou secondaire).
- les protéines antigéniques ne sont pas des glycoprotéines.

Afin de trancher entre ces trois possibilités, les Inventeurs ont utilisé une méthode de déglycosylation totale puis cherché des modifications de la masse ou de l'immunoréactivité des protéines antigéniques.

### 2) Déglycosylation

Deux types principaux de lien permettent l'association d'un oligosaccharide à un axe peptidique : la liaison O-glycosidique qui permet l'association d'un oligosaccharide à un résidu Sérine ou Thréonine (et rarement hydroxylysine) par l'intermédiaire d'une galactosamine N-acétylée et la liaison N-glycosidique qui rattache un oligosaccharide à un résidu asparagine par l'intermédiaire d'une glucosamine N-acétylée.

Pour déglycosyler les glycoprotéines, deux types de traitement peuvent être appliqués :
- des traitements aspécifiques permettant d'éliminer tous les types de radicaux glucidiques, qu'ils soient liés à la protéine par une liaison N- ou une liaison O-glycosidique. C'est le cas par exemple du traitement chimique par l'acide trifluorométhanesulfonique (TFMS);
- des traitements spécifiques de l'un ou l'autre de ces types de liaison. C'est le cas de la β-élimination par la soude pour les liaisons O-glycosidiques et d'un certain nombre d'hydrolyses enzymatiques plus ou moins spécifiques de la nature chimique du sucre N-lié pour les liaisons N-glycosidiques.

### a) Traitement aspécifique par le TFMS

L'analyse par immunotransfert après PAGE-SDS (7,5%) d'un extrait antigénique traité par le TFMS et d'un extrait incubé dans l'eau montre que les protéines antigéniques présentent une légère diminution de masse molaire apparente par rapport à l'échantillon témoin. Bien que faible cette différence d'environ 10 à 20 kD est observée pour toutes les conditions de traitement et retrouvée dans deux expériences distinctes. L'analyse en immunotransfert après IEF des protéines d'un extrait d'épithélium d'oesophage de rat traité par le TFMS montre que l'immunoréactivité spécifique des sérums de patients atteints de PR sur les échantillons témoins s'étale dans une zone de pHi d'environ 5,2 à 7,5. Après traitement par le TFMS, les protéines immunodétectées par les sérums de patients atteints de PR ont présenté des pHi plus basiques compris entre 6 et 8.

Ce changement de pHi et de masse molaire apparente des protéines antigéniques est en faveur d'une substitution glycanique des molécules. Cependant la persistance d'une mauvaise définition en PAGE-SDS et d'une large gamme de pHi en IEF suggère que les protéines antigéniques pourraient être substituées aussi par d'autres radicaux non glucidiques et/ou que les effets observés pourraient être dus au clivage d'une liaison peptidique particulièrement fragile.

Toutefois, la conservation de l'immunoréactivité après traitement par le TFMS est un argument fort en faveur de la nature non glucidique du ou des épitopes reconnus par les autoanticorps spécifiques de la PR.

### b) Traitement spécifique par la PNGase F

Afin de confirmer les résultats obtenus avec le TFMS, les Inventeurs ont entrepris des expériences de déglycosylation par l'enzyme PNGase F qui élimine spécifiquement les sucres N-liés.

La déglycosylation par la PGNase F a été effectuée sur un extrait antigénique brut après dialyse contre de l'eau et lyophilisation. L'analyse de cet extrait par immunodétection en PAGE-SDS et transfert ne fait apparaître aucune différence entre l'antigène témoin et l'antigène traité par la PNGase F. Deux interprétations de ce résultat sont possibles :
- l'antigène n'est pas substitué par des N-oligosaccharides,
- les oligosaccharides branchés à des résidus asparagine de la protéine ont une structure chimique originale qui les empêche d'être clivés par la PNGase F.

Cependant, une seule analyse monodimensionnelle ne permet pas d'affirmer clairement l'insensibilité à la PNGase F des protéines antigéniques puisque celles-ci se superposent partiellement, comme le montre l'analyse en électrophorèse bidimensionnelle.

### 3) Détection des sucres par des lectines

La réactivité éventuelle de l'antigène avec la lectine de germe de blé (WGA.) ou la concanavaline A (ConA) a été testée par "affinodétection" sur nitrocellulose et comparée aux profils d'immunodétection spécifiques des sérums de PR.

Les protéines de l'extrait antigénique brut d'oesophage de rat, reconnues après PAGE-SDS ou PAGE en conditions natives, par un sérum de patient atteint de PR, ont été différentes des protéines reconnues par les lectines. Cependant, des zones communes de réactivité, en particulier au niveau de la protéine de 210 kD en PAGE-SDS, suggère que la WGA et la ConA reconnaissent une partie de l'antigène. Des fractions antigéniques purifiées par chromatographie de gel filtration ont été analysées dans les mêmes conditions. Cette expérience a confirmé que des glycoprotéines de même poids moléculaire que certaines protéines antigéniques étaient reconnues par la ConA et que le profil de réactivité de la WGA semblait se rapprocher de celui d'un sérum de patient atteint de PR.

L'affinité des lectines vis-à-vis des protéines antigéniques d'oesophage de rat a été confirmée par analyse après IEF/PAGE-SDS de l'extrait antigénique brut.

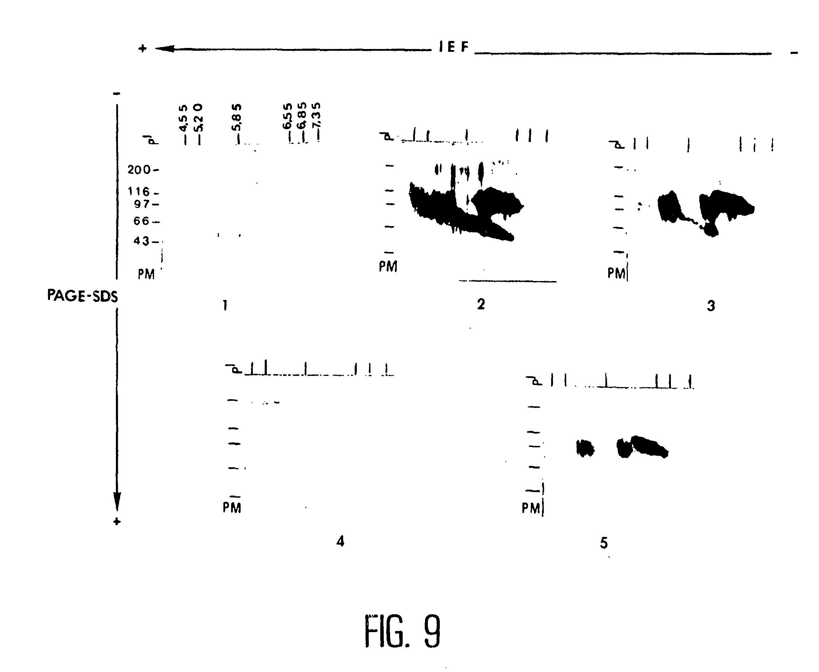

La figure 9 représente la détection des protéines d'un extrait antigénique brut, après électrophorèse bidimensionnelle (IEF, point isoélectrique 5 à 8/PAGE-SDS 7,5%) et transfert sur nitrocellulose, avec :
- en (1) : un sérum de patient atteint de PR, GIFOR -,
- en (2,3) : des sérums de patient atteint de PR, GIFOR +,
- en (4) : la ConA,
- en (5) : la WGA,
afin d'amplifier la réactivité, trois dépôts d'extrait antigénique ont été effectués sur le gel d'IEF. Une fraction de l'antigène (bande à 210 kD et zone immunoréactive entre 90 et 130 kD) déposée côté anode y a précipité, produisant une réactivité artéfactuelle dans la zone des pHi acides.

Cette étude a permis de montrer que les molécules reconnues par la ConA étaient différentes de l'antigène, mais que la WGA s'associait spécifiquement aux protéines antigéniques de 90-130 kD et 210 kD. Les protéines immunoréactives en forme de "virgule" s'étalant de 120 kD à 67 kD avec des pHi de 5 à 7,5 ne présentent, par contre, aucune réactivité vis-à-vis de la WGA.

### EXAMPLE 2 - CARACTERISATION DE L'ANTIGENE EXTRAIT DE L'EPIDERME HUMAIN

### I - MATERIEL ET METHODE

### A - PATIENTS ET SERUMS

La présente étude a été réalisée à partir de 64 sérums de patients atteints de diverses maladies rhumatologiques parfaitement caractérisées sur les plans clinique et biologique. Parmi ces 64 sérums, 45 proviennent de patients atteints d'une PR définie selon les critères de l'"American Rheumatism Association". 32 sérums provenant de sujets sains, hommes et femmes, ont été utilisés à titre de contrôle.

Deux anticorps monoclonaux (AcM), l'un spécifique des cytokératines humaines dénommé F 12-19 (Anti-cytokeratin Ref. 220-81, Dept. Biosoft CLONATEC), l'autre spécifique de la filaggrine humaine dénommé AKH-1 (Réf. catalogue BT - 576, Biomedical Technologies Inc.) ont été utilisés.

### B - EXTRACTION DES PROTEINES EPIDERMIQUES

Des fragments de peau recueillis après chirurgie plastique mammaire et abdominale ou après circoncision ont été conservés à -80°C. L'épiderme a été séparé mécaniquement du derme, selon la méthode décrite par Kassis et Sondergaard (ARCH. DERMATOL. RES. 273 : 301-306, 1982), par un traitement thermique à 4°C puis à 57°C en tampon PBS (KH₂/K₂H-PO₄ 8,5 mM ; NaCl 150 mM) contenant de l'EDTA 5 mM et du PMSF 0,5 mM, puis broyé mécaniquement en tampon Tris-HCl 40 mM pH 7,4 ; NaCl 150 mM ; EDTA 10 mM ; NP40 0,5%, ci-après dénommé tampon A.

Après centrifugation à 12 000 g pendant 15 minutes, le surnageant a été séparé du culot contenant les cytokératines épidermiques.

En outre, d'autres extractions ont été réalisées selon le même protocole mais dans un tampon Tris-HCl 20 mM pH 7,4 ; PMSF 0,5 mM, ci-après dénommé tampon B.

Les différentes fractions ont été conservées à -20°C.

### C - ELECTROPHORESES

Les protéines des diverses fractions ont été analysées par IEF à l'équilibre ou avant l'équilibre (NEpHGE), ou par PAGE-SDS ou PAGE en conditions natives. Des électrophorèses bidimensionnelles ont également été réalisées.

Les protéines ont parfois été précipitées par 4 volumes d'éthanol ou par le TCA à 15% final, afin d'en augmenter la concentration.

### D - IMMUNODETECTION

Les protéines ont été transférées sur membrane de nitrocellulose selon la technique décrite par TOWBIN et al. (PNAS 76 : 4350-4354, 1979). Les sérums ont été utilisés dilués du 1/10 au 1/100 après incubation des bandelettes de nitrocellulose en PBS, Tween-20 0,05%. L'incubation en présence des sérums a été poursuivie pendant 1 heure à 37°C puis 12 heures à 4°C.

Les anticorps monoclonaux AKH-1 (dilué au 1/100) ou F12-19 (dilué au 1/500) ont été incubés pendant 2 heures à 37°C. Après lavages, les bandelettes de nitrocellulose ont été révélées par des anticorps secondaires, anti-IgG humaines ou murines, marqués à la peroxydase.

### E - TRAITEMENTS ENZYMATIQUES

L'extrait épidermique concentré (80 g/ l) a été traité par la protéinase K (1 mg/ml, 30 minutes, 37°C en présence de SDS 1%) ou par la nucléase de microccoque (100 U/ml, 30 minutes, 37°C dans un tampon Tris-HCl 30 mM, CaCl₂ 5mM, pH8).

### F - IMMUNOPRECIPITATION

L'extrait épidermique préadsorbé sur Protéine A-Sépharose a été incubé à 37°C pendant 2h en présence de l'anticorps AKH-1 ou F12-19 et de NaCl, 1 M final.

Les complexes anticorps-antigène formés ont été ensuite recueillis par la protéine A-Sépharose puis analysés par immunotransfert après PAGE-SDS.

### G - PURIFICATION DES CYTOKERATINES

Les cytokératines uréosolubles ont été purifiées à partir d'épiderme mammaire selon la méthode décrite par T.T SUN et H. GREEN (J. Biol. Chem. 252 : 2053-2060, 1978).

### H - PURIFICATION DE LA FILAGGRINE HUMAINE

La filaggrine a été extraite à partir de peau mammaire selon la technique décrite par LYNLEY et DALE (B.B.A 774 : 28-35, 1983) avec les modifications suivantes : l'extrait épidermique en urée 6M, préparé en absence d'agent réducteur, a été purifié sur chromatographie FPLC avec une résine échangeuse d'anions. Les protéines cationiques non retenues ont été précipitées par 4 volumes d'acétone, redissoutes dans un petit volume de tampon contenant 1,5% de SDS et 2,5% de 2-ME et déposées au sommet d'un gel préparatif PAGE-SDS de 10 cm de hauteur. Ce gel a ensuite été transféré sur une membrane du type Immobilon^{(R)}-PVDF commercialisée par la Société MILLIPORE. La zone correspondant à la filaggrine a été repérée par immunodétection avec l'anticorps AKH-1 et découpée ; la filaggrine a alors été éluée par 0,3 ml/cm² de tampon d'élution (Tris-HCl 50 mM pH 8,8 ; SDS 2% ; Triton X-100 1%) et concentrée par précipitation.

### I - PURIFICATION DE L'ANTIGENE

20 cm² d'épiderme mammaire ont été broyés au potter électrique dans le tampon A. L'homogénat a été centrifugé pendant 15 minutes à 10 000 g et le surnageant précipité par le TCA (15% final). Le précipité a été re-solubilisé dans un petit volume de Tris-HCl 30 mM pH8, CaCl₂ 5mM ; la fraction soluble a été clarifiée par centrifugation, additionnée de SDS 1,5% et 2ME 2,5% et déposée au sommet d'un gel préparatif SDS de 10 cm de hauteur. L'antigène a alors été purifié, par élution, comme décrit ci-dessus pour la filaggrine, la zone correspondant à l'antigène étant repérée par immunodétection avec un sérum de PR.

### II - RESULTATS

### A - CARACTERISATION PHYSICO-CHIMIQUE ET BIOCHIMIQUE DE L'ANTIGENE

Afin de caractériser l'antigène reconnu par les sérums de PR, un extrait épidermique mammaire a été préparé par homogénéisation d'épiderme mammaire dans le tampon A, en présence d'un détergent non ionique (NP40). Cet extrait soluble a été analysé par immunodétection après PAGE et transfert des protéines sur nitrocellulose.

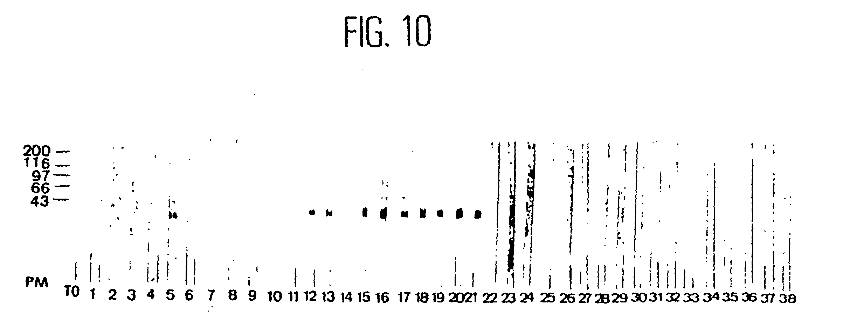

La figure 10 représente l'analyse en immunotransfert de l'extrait NP40 précédent avec des sérums de PR et des sérums de contrôle. L'extrait NP40 (8 mg/ml) a été précipité par TCA, repris en tampon Tris-CaCl₂ en le concentrant 7 fois puis analysé par immunotransfert après PAGE-SDS (gradient 8-25%). Les sérums ont été dilués au 1/10 en PBS-Tween 20 à 0,05%. Les sérums 1 et 3 proviennent de patients atteints de myélome malin, le sérum 2 provient d'un patient atteint de maladie de SHARP, le sérum 4 provient d'un patient atteint d'arthrose, le sérum 5 provient d'un patient atteint de polymyosite, le sérum 6 provient d'un patient atteint de maladie de PAGET, les sérums 7 à 21 proviennent de patients atteints de PR et les sérums 22 à 38 proviennent de sujets sains ; "T₀" est un témoin négatif incubé seulement avec l'anticorps anti-Ig humaines, marqué.

La plupart des sérums de patients atteints de PR (80%) ont reconnu une protéine présentant en PAGE-SDS un poids moléculaire d'environ 40 kD. Sa migration n'a pas été modifiée par l'omission de l'agent réducteur (2-ME) dans le tampon d'échantillon, ce qui indique que cette molécule ne contient probablement pas de pont disulfure. Au contraire, 89% des sérums de patients atteints d'une maladie rhumatologique non-rhumatoide et aucun des sérums de contrôle n'ont reconnu cet antigène. Une protéine de 68 kD a été détectée non spécifiquement par une large proportion de sérums appartenant à tous les groupes testés et donc vraisemblablement par des auto-anticorps naturels.

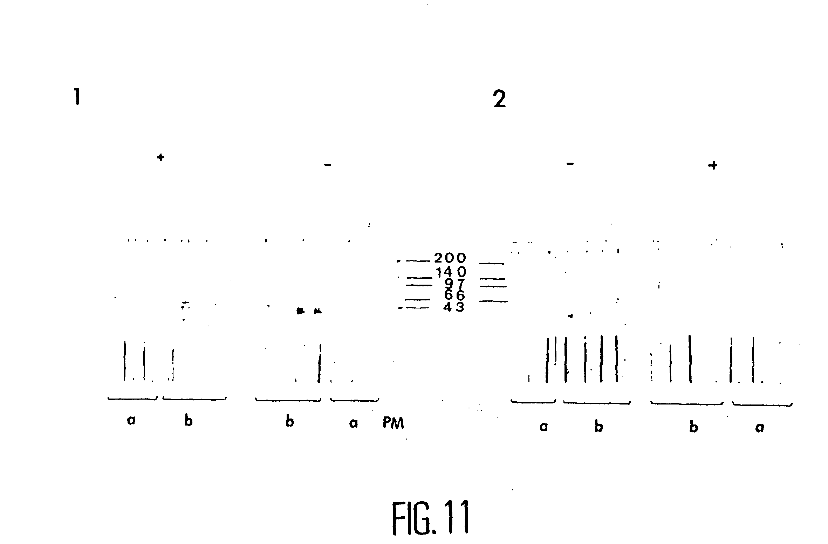

La figure 11 représente l'analyse en immunotransfert de l'extrait antigénique NP40 précédent, traité ou non par une nucléase (1) ou une protéase (2). En (1), l'extrait NP40 d'épiderme mammaire a été précipité par le TCA, repris en tampon Tris-CaCl₂ puis traité avec (+) ou sans (-) nucléase microccocale (100 U/ml) pendant 30 minutes à 37°C. En (2), un extrait NP40 d'épiderme humain identique au précédent a été précipité par le TCA puis repris dans le SDS 1% et traité sans (-) ou avec (+) 10 mg/ml de protéinase K pendant 30 minutes à 37°C. Dans tous les cas, les protéines ont été séparées en PAGE-SDS (8-25%), transférées puis immunodétectées avec des sérums de contrôle (a) et des sérums de patients atteints de PR (b) dilués au 1/100. La disparition de la région immunoréactive après un traitement de l'extrait par la protéinase K confirme la nature protéique de la molécule porteuse du ou des épitopes reconnus par les auto-anticorps, alors que cette région n'a pas été modifiée après une digestion des acides nucléiques par la nucléase de Microcoque.

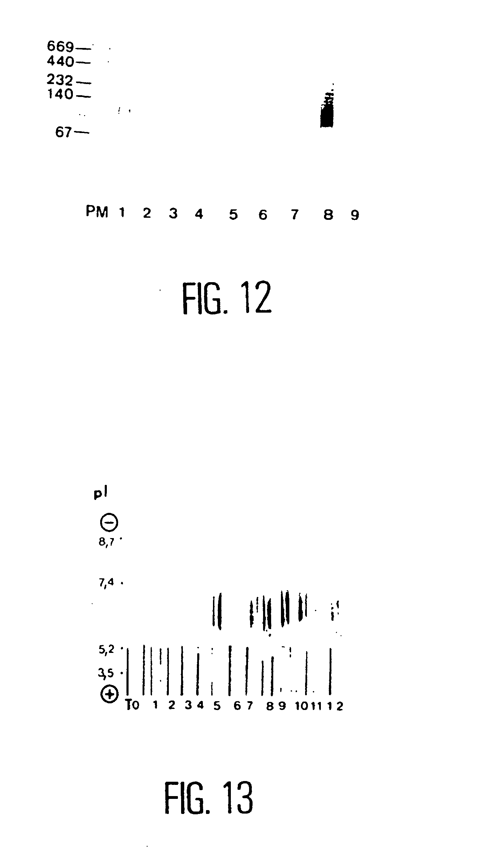

La figure 12 représente l'analyse de l'antigène par électrophorèse en conditions natives ; cette analyse a consisté à précipiter l'extrait NP40 d'épiderme humain par l'éthanol, puis à effectuer une immunodétection après PAGE en conditions natives (8-25%) et transfert sur nitrocellulose avec des sérums de patients atteints de PR (pistes 1-4 et 8-9) et de sérums témoins (pistes 5-7), dilués au 1/100. L'antigène humain se sépare en plusieurs protéines antigéniques dont la migration est similaire à celle de témoins dont les poids moléculaires sont compris entre 80 et 400 kD, les protéines les plus immunoréactives se situant dans les zones comprises entre 80 et 120 kD.

La figure 13 représente l'analyse de l'antigène par IEF. Les protéines de l'extrait NP40 d'épiderme mammaire ont été précipitées par l'éthanol, séparées par IEF (pHi 3 à 9), transférées sur nitrocellulose puis immunodétectées avec des sérums de patients atteints de PR (pistes 5-12) et des sérums témoins (pistes 1-4) dilués au 1/100. L'antigène humain présente une distribution très hétérogène de pHi, ceux-ci variant de 5,8 à 7,4. Cette hétérogénéité de pHi a été vérifiée en électrophorèse bidimensionnelle (IEF/PAGE-SDS). La protéine reconnue spécifiquement par les sérums de patient atteints de PR, après transfert d'un tel gel, montre une image caractéristique en forme de "virgule", le poids moléculaire apparent diminuant au fur et à mesure que le pHi devient plus basique.

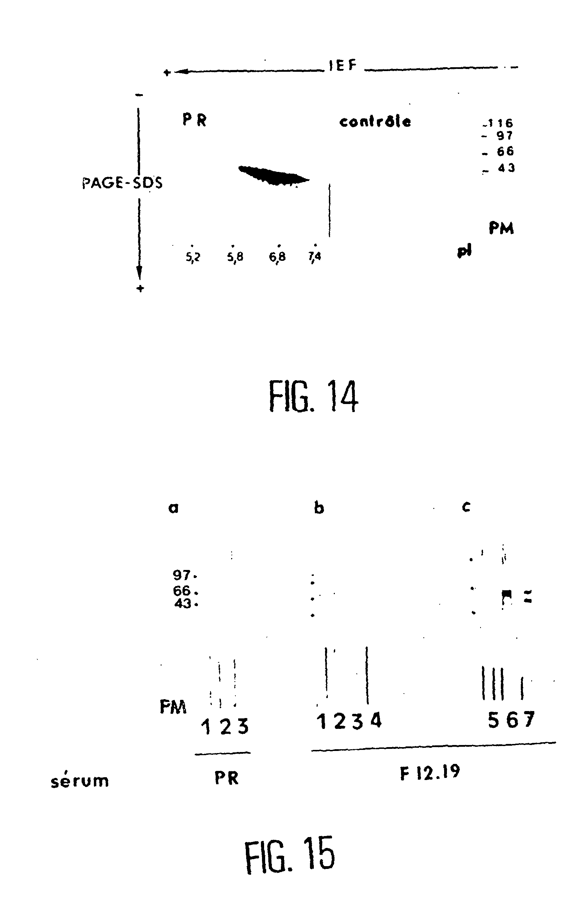

La figure 14 représente l'analyse de l'antigène en gel bidimensionnel IEF/PAGE-SDS ; les protéines de l'extrait NP40 d'épiderme mammaire ont été précipitées par l'éthanol, séparées par IEF (pHi 5 à 8) suivie de PAGE-SDS (12,5%), transférées sur nitrocellulose et immunodétectées par un sérum de patient atteint de PR et un sérum de contrôle.

L'hétérogénéité marquée de l'antigène, aussi bien en masse qu'en pHi, indique probablement la présence d'isoformes modifiées ; elle suggère qu'il pourrait être glycosylé et/ou phosphorylé. Cependant un traitement par la peptide N-glycosidase-F n'a modifié ni sa migration ni son immunoréactivité après PAGE-SDS.

### B - HYDROSOLUBILITE DE L'ANTIGENE

5 cm² d'épiderme mammaire ont été broyés mécaniquement dans le tampon A ou dans le tampon B. Les homogénats ont ensuite été centrifugés 10 minutes à 10 000 g et une quantité égale des protéines solubles des deux surnageants a été précipitée par l'éthanol puis séparée par PAGE en conditions natives (8-25%), transférée sur nitrocellulose puis immunodétectée avec des sérums de patients atteints de PR et des sérums de contrôle. Cette étude indique que l'antigène est extrait non seulement en présence d'un détergent (tampon A) mais également en son absence (tampon B). En outre, l'antigène est toujours retrouvé majoritairement dans les fractions solubles et présents dans les culots en très faible quantité. Ces résultats indiquent que l'antigène humain, comme celui d'oesophage de rat, est une molécule très hydrosoluble.

### C - EXPRESSION DE L'ANTIGENE DANS DIFFERENTS TERRITOIRES ANATOMIQUES EPIDERMIQUES

L'analyse par immunotransfert des protéines d'un extrait NP40 d'épiderme abdominal et d'épiderme préputial indique que l'antigène associé à la PR est présent dans l'épiderme humain indépendamment du sexe et du territoire anatomique (sein, abdomen ou prépuce), ainsi que de l'âge (enfants et adultes). L'antigène étant histologiquement localisé dans le *Stratum Corneum*, il est légitime de penser que son abondance dépendra de l'épaisseur de celui-ci qui varie suivant le territoire anatomique.

### D - IDENTIFICATION DE L'ANTIGENE

L'antigène reconnu par les sérums de malades atteints de PR n'est pas une cytokératine épidermique humaine.

La figure 15 représente :
- en (a) : un extrait NP40 d'épiderme mammaire immunodétecté, après PAGE-SDS (8-25%) et transfert sur nitrocellulose, avec un sérum de patient atteint de PR, préincubé d'une part avec le tampon d'extraction (piste 1), avec l'extrait lui-même (piste 2) et avec une fraction insoluble enrichie en cytokératines épidermiques (piste 3).
- en (b) : les cytokératines uréosolubles immunodétectées, après PAGE-SDS (8-25%) et transfert, avec l'AcM anti-cytokératines F12-19 préincubé soit avec le tampon d'extraction (piste 1), soit avec l'extrait épidermique (piste 2), soit avec une fraction enrichie en cytokératines (piste 3), soit avec les cytokératines uréosolubles (piste 4).
- en (c) : les protéines de l'extrait d'épiderme (piste 5), de la fraction enrichie en cytokératines (piste 6) et les cytokératines uréosolubles pures (piste 7) séparées par PAGE-SDS (8-25%), transférées et immunodétectées avec l'AcM anti-cytokératines F12-19.

L'immunoréactivité des sérums de patients atteints de PR n'a pas été inhibée par une préincubation de ceux-ci sur des cytokératines épidermiques (enrichies ou purifiées) alors qu'elle a disparu en présence d'extrait hydrosoluble de peau. De même, l'anticorps monoclonal F12-19 n'a reconnu en immunotransfert, aucune protéine de l'extrait antigénique.

La forme caractéristique en virgule de l'antigène en gel bidimensionnel et son poids moléculaire apparent en PAGE-SDS sont voisins de ceux de la filaggrine, protéine qui apparaît dans le *Stratum granulosum* au cours de la différenciation épidermique (SARRET et al., Path. Biol. 37:297-303, 1989). Les Inventeurs ont donc cherché à déterminer si la filaggrine et l'antigène de l'invention étaient identiques et si les sérums de malades atteints de PR contenaient des autoanticorps anti-filaggrine.

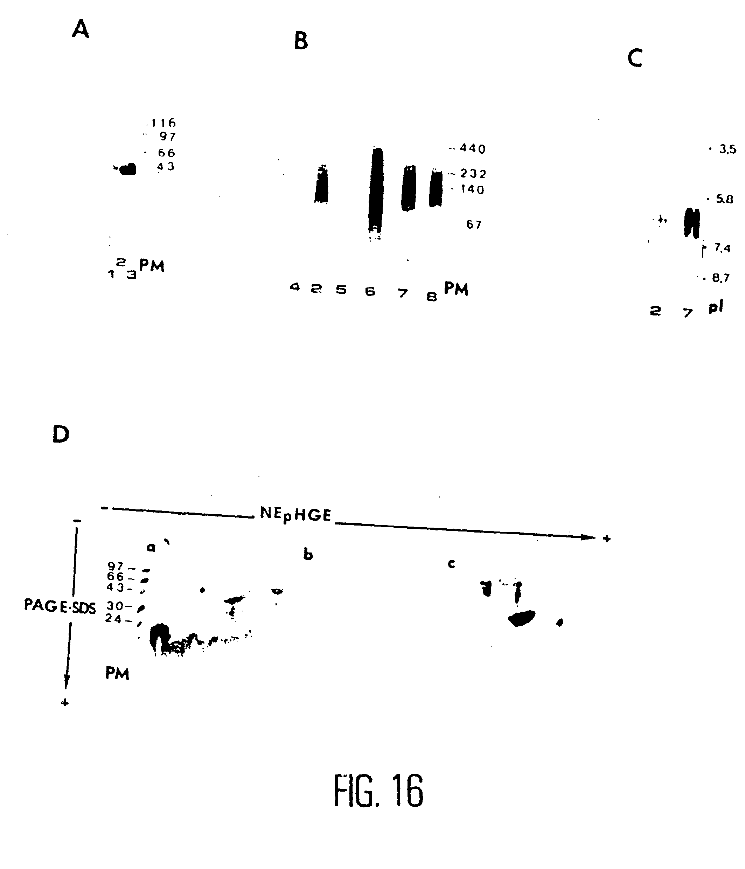

La figure 16 représente l'analyse d'un immunotransfert des protéines d'un extrait d'épiderme mammaire séparées en (A) par PAGE-SDS (12,5%), en (B) par PAGE en conditions natives et en (C) par IEF (pHi 3 à 9), puis transférées sur nitrocellulose et immunodétectées par des sérums de patients atteints de PR (pistes 1, 3, 6, 7 et 8), l'AcM anti-filaggrine AKH-1 (piste 2), un AcM de contrôle (piste 4) et un sérum de contrôle (piste 5). Ces protéines ont été séparées en triplica (D) par NEpHGE suivi de PAGE-SDS (8-25%) puis transférées sur nitrocellulose et colorées au Rouge Ponceau (a) ou immunodétectées par l'anticorps monoclonal AKH-1 (b), et par un sérum de patient atteint de PR (c). L'antigène est reconnaissable à sa forme caractéristique en "virgule".

L'anticorps monoclonal anti-filaggrine humaine AKH-1 reconnaît spécifiquement en immunotransfert une protéine épidermique humaine soluble en présence de NP40, présentant les mêmes caractéristiques de migration en conditions d'électrophorèse dénaturante ou native que l'antigène de la PR. Après IEF et gel bidimensionnel, l'antigène et la protéine reconnus en immunotransfert par AKH-1 présentent une image identique.

Les Inventeurs ont alors cherché à déterminer si l'anticorps monoclonal AKH-1 pouvait immunoprécipiter l'antigène à partir de l'extrait épidermique en NP40.

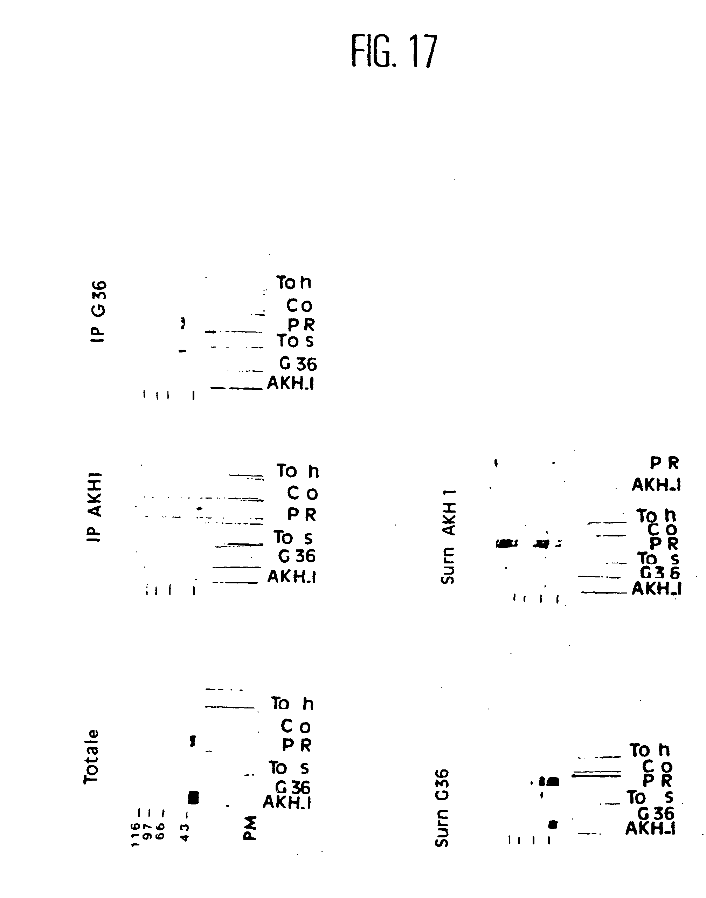

La figure 17 présente l'analyse comparative des protéines d'un extrait épidermique NP40, préadsorbé sur protéine A-Sépharose, les protéines étant séparées par PAGE-SDS (12,5%) avant immunoprécipitation avec l'AcM AKH-1 (représentées par "Totale" à la figure 17) ou après immunoprécipitation avec l'AcM AKH-1 (représentées par "IP AKH-1" à la figure 17), ou avec l'AcM de contrôle G36 (représentées par "IP G36" à la figure 17). Après transfert sur nitrocellulose, ces protéines ont été immunodétectées avec les anticorps AKH-1, G36, un sérum de patient atteint de PR (PR), un sérum de contrôle (CO) ou avec les seuls anticorps secondaires anti-Ig de souris (T₀ S) et anti-Ig humaines (T₀ h). Les protéines présentes dans les surnageants des fractions immunoprécipitées avec G36 (représentées par "Surn G36" à la figure 17) et AKH-1 (représentées par "Surn AKH-1" à la figure 17) ont été analysées de la même façon après précipitation par le TCA.

Ces résultats montrent que AKH-1 a effectivement immunoprécipité une protéine épidermique de même poids moléculaire que l'antigène de la PR, cette molécule étant d'ailleurs reconnue spécifiquement en immunotransfert par les sérums de patients atteints de PR. Cette immunoprécipitation a été réalisée en présence de NaCl 1 M, ce qui rend peu probable une co-immunoprécipitation dûe à une association entre les deux protéines (filaggrine et antigène). Afin de vérifier que les protéines de l'extrait reconnu par AKH-1 et par les sérums de patient atteint de PR étaient les mêmes, les surnageants d'une immunoprécipitation par l'AcM anti-filaggrine ont été subséquemment analysés par immunotransfert avec un sérum de patients atteints de PR. La figure 17 montre que l'extrait a été épuisé (partiellement ou totalement) en antigène et en filaggrine par l'AcM anti-filaggrine. A l'inverse, les sérums de patients atteints de PR n'ont pas immunoprécipité l'antigène. Ce résultat négatif peut être dû à une faible affinité des autoanticorps pour l'antigène, à une trop faible quantité d'autoanticorps dans le sérum ou à une non reconnaissance par ceux-ci de l'antigène sous sa forme native. Cette dernière hypothèse est peu probable puisque l'antigène est immunodétectable sur nitrocellulose après électrophorèse en conditions natives.

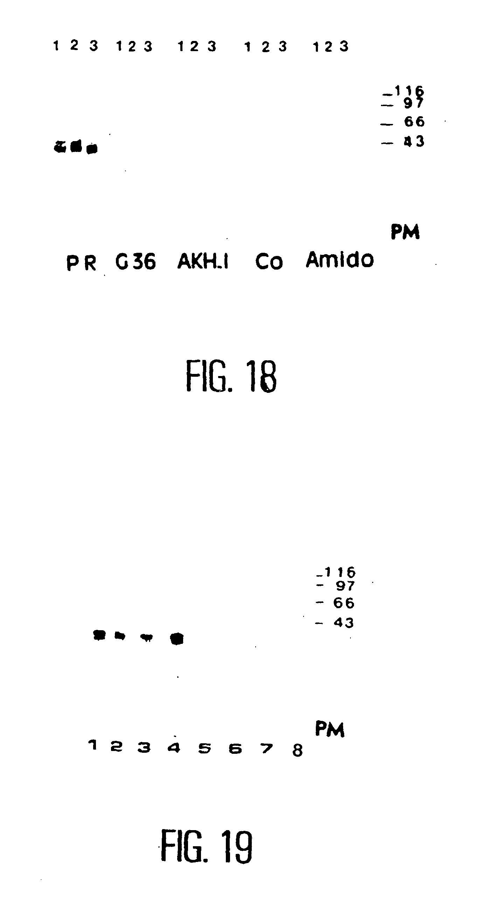

Afin de confirmer que la filaggrine et l'antigène de la PR ont au moins en commun l'épitope détecté par l'AcM AKH-1, l'antigène a été purifié par élution à partir d'une membrane de transfert. La figure 18 représente l'analyse par PAGE-SDS et immunotransfert des protéines des diverses fractions au cours de la purification. Les protéines totales d'un extrait d'épiderme humain (piste 1), les protéines solubles après précipitation de l'extrait par le TCA (piste 2) et celles de la fraction antigénique purifiée finale (piste 3) ont été séparées par PAGE-SDS (12,5%), transférées sur nitrocellulose et colorées à l'amido-black (Amido) ou immunodétectées avec un sérum de patient atteint de PR (PR), un sérum de contrôle (CO), un AcM anti-filaggrine (AKH-1) et un AcM de contrôle (G36).

Les protéines majeures, solubles après précipitation au TCA, apparaissent sous la forme d'un doublet de poids moléculaire (PM) apparent d'environ 40 kD. Ce doublet est la principale bande immunoréactive reconnue par un sérum de patient atteint de PR. Cette protéine a alors été purifiée à partir d'un gel PAGE-SDS préparatif par transfert puis élution. L'antigène de la PR a été spécifiquement immunodétecté après PAGE-SDS, par l'AcM anti-filaggrine AKH-1.

De façon à vérifier que les sérums de patients atteints de PR reconnaissaient également la filaggrine humaine et donc que celle-ci était étroitement apparentée à l'antigène, les Inventeurs ont purifié la filaggrine selon la méthode décrite par LINLEY et DALE (BBA 744:28-35, 1983). La figure 19 représente l'analyse de la filaggrine humaine purifiée, analysée par PAGE-SDS (12,5%), transférée sur nitrocellulose et immunodétectée avec des sérums de patients atteints de PR (pistes 1-4), un sérum de contrôle (piste 5), un AcM de contrôle (piste 6) et l'AcM AKH-1 (piste 7) ou colorée par le Rouge Ponceau (piste 8). La figure 19 montre que les sérums de patients atteints de PR reconnaissent spécifiquement la filaggrine humaine.

## Revendications

1. Antigène hydrosoluble reconnu par des autoanticorps spécifiquement présents chez les patients atteints de polyarthrite rhumatoïde (PR), **caractérisé en ce qu'**il présente des réactions antigéniques croisées avec la filaggrine et la profilaggrine humaine, et **en ce qu'**il est choisi parmi :
a) un antigène protéique susceptible d'être obtenu à partir d'épithélium oesophagien de rat, et possédant les propriétés suivantes :
- après électrophorèse en gel de polyacrylamide-SDS, il se sépare en deux fractions antigéniques :
i) une fraction de poids moléculaire apparent moyen d'environ 210 kD,
ii) une fraction de poids moléculaire apparent moyen compris entre environ 90 et environ 130 kD,
- après électrophorèse en gel de polyacrylamide en conditions natives, il se sépare en trois fractions antigéniques :
iii) une fraction qui migre comme la ferritine de rate de cheval (poids moléculaire : 440 kD)
iv) une fraction qui migre comme la catalase de foie de boeuf (poids moléculaire : 232 kD),
v) une fraction qui migre entre la lactico-déshydrogénase de coeur de boeuf, et la sérum-albumine bovine (poids moléculaires respectifs : 140 kD et 67 kD).
- après électrophorèse bidimensionnelle par isoélectrofocalisation, suivie d'une électrophorèse en gel de polyacrylamide-SDS, il se sépare en trois fractions antigéniques :
vi) une fraction dont le poids moléculaire apparent moyen est d'environ 210 kD, et dont le pHi est d'environ 5,85 à environ 6,85 ;
vii) une fraction dont le poids moléculaire apparent moyen est d'environ 130 à environ 90 kD, et dont le pHi est d'environ 5,85 à environ 7,35 ;
viii) une fraction dont le poids moléculaire apparent moyen est d'environ 120 à environ 67 kD, et dont le pHi est d'environ 5 à environ 7,5 ;
b) l'une quelconque des fractions i) à viii) de l'antigène a) défini ci-dessus ;
c) un antigène protéique susceptible d'être obtenu à partir de l'épiderme humain, et qui, après électrophorèse bidimensionnelle par isoélectrofocalisation, suivie d'une électrophorèse en gel de polyacrylamide-SDS, présente un poids moléculaire apparent moyen d'environ 40 kD, et dont le pHi est d'environ 5,8 à environ 7,4.

2. Utilisation d'un antigène choisi parmi :
- un antigène selon la revendication 1 ;
- la filaggrine ou la profilaggrine humaine ;
pour le diagnostic *in vitro* de la polyarthrite rhumatoïde.

3. Procédé de diagnostic *in vitro* de la polyarthrite rhumatoïde dans un échantillon biologique **caractérisé en ce qu'**il comprend au moins les étapes suivantes :
- la mise en contact de cet échantillon biologique avec au moins un antigène selon la revendication 1, ou avec une préparation de filaggrine ou de profilaggrine humaine éventuellement marquée, ou encore leur conjugué avec une molécule porteuse, dans des conditions permettant la formation d'un complexe immunologique avec les auto-anticorps spécifiques de la PR éventuellement présents dans ledit échantillon ;
- la détection dudit complexe immunologique.

4. Nécessaire pour le diagnostic *in vitro* de la polyarthrite rhumatoïde à partir d'un échantillon biologique, **caractérisé en ce qu'**il comprend :
- au moins un antigène selon la revendication 1 ou au moins une préparation de filaggrine ou de profilaggrine, éventuellement marqués, ou conjugués avec une molécule porteuse ;
- des réactifs pour la constitution d'un milieu propice à la réaction immunologique avec les autoanticorps éventuellement présents dans ledit échantillon biologique ;
- un ou plusieurs réactifs pour la détection du complexe immunologique formé ;
- le cas échéant, des échantillons de référence, tels que sérum(s) négatif(s) et/ou sérum(s) positif(s).

5. Procédé de purification d'autoanticorps spécifiquement présents chez des patients atteints de polyarthrite rhumatoïde, **caractérisé en ce qu'**il comprend au moins les étapes suivantes :
- la mise en contact d'un fluide biologique d'un patient atteint de polyarthrite rhumatoïde avec au moins un antigène selon la revendication 1, ou avec la filaggrine ou la profilaggrine humaine, dans des conditions permettant la formation de complexes immunologiques avec les autoanticorps présents dans ledit fluide biologique,
- l'isolement desdits complexes du milieu réactionnel et/ou la séparation des autoanticorps desdits complexes immunologiques.

6. Procédé de préparation d'anticorps **caractérisé en ce qu'**on utilise comme immunogène au moins un antigène selon la revendication 1.

7. Procédé de préparation d'anticorps **caractérisé en ce qu'**on utilise comme antigène d'immunisation la filaggrine ou la profilaggrine humaine, et **en ce qu'**on recueille les anticorps reconnaissant également un antigène de l'épithélium oesophagien de rat, tel que défini dans la revendication 1.

8. Procédé de préparation d'anticorps anti-idiotypes, lequel procédé est **caractérisé en ce qu'**il comprend une étape au cours de laquelle on immunise un animal avec un anticorps monoclonal ou polyclonal obtenu par un procédé selon la revendication 7.

9. Préparation d'anticorps polyclonaux **caractérisée en ce qu'**elle est susceptible d'être obtenue par le procédé selon la revendication 6.

10. Préparation d'anticorps **caractérisée en ce qu'**elle est susceptible d'être obtenue par le procédé selon la revendication 7.

## Patentansprüche

1. Wasserlösliches Antigen, welches durch Autoantikörper erkannt wird, die spezifisch in an rheumatoider Polyarthritis (PR) erkrankten Patienten vorliegen, **dadurch gekennzeichnet, dass** das Antigen antigene Kreuzreaktionen mit menschlichem Filaggrin oder Profilaggrin zeigt und dass es ausgewählt ist aus:
a) einem vom Osophagusepithel der Ratte erhältlichen Eiweißantigen mit folgenden Eigenschaften:
- das Antigen trennt sich nach einer SDS-Polyacrylamidgelelektrophrese in zwei Antigenfraktionen:
i) eine Fraktion mit einem mittleren Molekulargewicht von ungefähr 210 kD;
ii) eine Fraktion mit einem mittleren Molekulargewicht von ungefähr 90 bis ungefähr 130 kD;
- das Antigen trennt sich nach einer Polyacrylamidgelelektrophorese unter nativen Bedingungen in drei Antigenfraktionen:
iii) eine Fraktion, die mit der Geschwindigkeit von Pferdeferritin wandert (Molekulargewicht: 440 kD);
iv) eine Fraktion, die mit der Geschwindigkeit von Rinderleberkatalase wandert (Molekulargewicht: 232 kD);
v) eine Fraktion, die mit einer Geschwindigkeit zwischen der von Rinderherzlacticodehydrogenase und der von bovinem Serumalbumin wandert (jeweiliges Molekulargewicht: 140 kD und 67 kD);
- das Antigen trennt sich nach einer zweidimensionalen Elektrophorese mittels isoelektrischer Fokussierung, gefolgt von einer SDS-Polyacrylamidgelelektrophorese in drei Antigenfraktionen:
vi) eine Fraktion, deren mittleres Molekulargewicht ungefähr 210 kD ist und deren pKi von ungefähr 5,85 bis ungefähr 6,85 ist;
vii) eine Fraktion, deren mittleres Molekulargewicht von ungefähr 130 bis ungefähr 90 kD ist und dessen pHi von ungefähr 5,85 bis ungefähr 7,35 ist;
viii) eine Fraktion, deren mittleres Molekulargewicht von ungefähr 120 bis ungefähr 67 kD ist und deren pHi von ungefähr 5 bis ungefähr 7,5 ist;
b) einer beliebigen der Fraktionen i) bis viii) des oben definierten Antigens a);
c) einem aus der menschlichen Epidermis erhältlichen Eiweisantigen, welches nach einer zweidimensionalen Elektrophorese mittels isoelektrischer Fokussierung, gefolgt von einer SDS-Polyacrylamidgelelektrophorese ein mittleres Molekulargewicht von ungefähr 40 kD zeigt und dessen pHi von ungefähr 5,8 bis ungefähr 7,4 ist.

2. Verwendung eines Antigens ausgewählt aus:
- einem Antigen nach Anspruch 1;
- menschlichem Filaggrin oder Profilaggrin;
zur in vitro Diagnose von rheumatoider Polyarthritis.

3. Verfahren zur *in vitro* Diagnose von rheumatoider Polyarthritis in einer biologischen Probe, **dadurch gekennzeichnet, dass** es wenigstens einen der folgenden Verfahrensschritte aufweist:
Kontaktieren der biologischen Probe mit wenigstens einem Antigen nach Anspruch 1 oder einer Zubereitung von menschlichem Filaggrin oder Profilaggrin, wobei das Filaggrin oder Profilaggrin optional markiert ist, oder ferner deren Konjugat mit einem Trägermolekül, unter Bedingungen, welche die Bildung eines immunologischen Komplexes mit den Autoantikörpern erlauben, die für eine eventuell in der Probe vorliegende PR spezifisch sind;
Nachweis des genannten immunologischen Komplexes.

4. Set zur *in vitro* Diagnose von rheumatoider Polyarthritis in einer biologischen Probe, **dadurch gekennzeichnet, dass** das Set umfasst:
wenigstens ein Antigen nach Anspruch 1 oder wenigstens eine Zubereitung von, optional markiertem, Filaggrin oder Profilaggrin, oder von Konjugaten, mit einem Trägermolekül;
Reagenzien zur Ausbildung eines für die immunologische Reaktion mit den möglicherweise in der biologischen Probe vorhandenen Autoantikörpem günstigen Milieus;
ein oder mehrere Reagenzien zum Nachweis des gebildeten immunologischen Komplexes;
ggf. Vergleichsproben, welche serumnegativ und/oder serumpositiv sind.

5. Verfahren zur Reinigung von Autoantikörpern, welche spezifisch bei an rheumatoider Polyarthritis erkrankten Patienten vorliegen, **dadurch gekennzeichnet, dass** es wenigstens die folgenden Verfahrensschritte aufweist:
Kontaktieren einer biologischen Flüssigkeit eines an rheumatoider Polyarthritis erkrankten Patienten mit wenigstens einem Antigen nach Anspruch 1 oder mit menschlichem Filaggrin oder Profilaggrin unter Bedingungen, welche die Bildung von immunologischen Komplexen mit den in der biologischen Flüssigkeit vorliegenden Autoantikörpern erlauben,
Isolierung der genannten Komplexe aus dem Reaktionsmilieu und/oder Trennung der Autoantikörper der genannten immunologischen Komplexe.

6. Verfahren zur Herstellung von Antikörpern, **dadurch gekennzeichnet, dass** man als Immunogen wenigstens ein Antigen nach Anspruch 1 verwendet.

7. Verfahren zur Herstellung von Antikörpern, **dadurch gekennzeichnet, dass** man als Immunisierungsantigen menschliches Filaggrin oder Profilaggrin verwendet und dass die wiedererkannten Antikörper gesammelt werden, die gleichermaßen ein Antigen des Ösophagusepithels der Ratte erkennen, wie in Anspruch 1 definiert ist.

8. Verfahren zur Herstellung von antiiodotypischen Antikörpern, wobei das Verfahren **dadurch gekennzeichnet ist, dass** es einen Schritt umfasst, im Verlauf dessen ein Tier mit einem nach einem Verfahren gemäß Anspruch 7 erhaltenen monoklonalen oder polyklonalen Antikörper immunisiert wird.

9. Zubereitung von polyklonalen Antikörpern, **dadurch gekennzeichnet, dass** sie nach einem Verfahren nach Anspruch 6 erhältlich ist.

10. Antikörperzubereitung, **dadurch gekennzeichnet, dass** sie nach einem Verfahren nach Anspruch 7 erhältlich ist.

## Claims

1. A water-soluble antigen recognized by autoantibodies specifically present in patients suffering from rheumatoid arthritis (RA), **characterized in that** said antigen exhibits antigenic cross-reactions with human profilaggrin and filaggrin, and **in that** it is selected among:
a) a protein antigen capable of being extracted from rat oesophageal epithelium, and possessing the following properties:
- after SDS-polyacrylamide gel electrophoresis, it separates into the following two antigenic fractions:
i) a fraction of apparent average molecular weight of approximately 210 kD,
ii) a fraction of apparent average molecular weightbetween approximately 90 and 130 kD,
- after polyacrylamide gel electrophoresis under native conditions, it separates into the following three antigenic proteins:
iii) a fraction which migrates like horse spleen ferritin (molecular weight: 440 kD),
iv) a fraction which migrates like ox liver catalase (molecular weight: 232 kD),
v) a fraction which migrates between ox heart lactic dehydrogenase, and bovine serum albumin (respective molecular weights: 140 kD and 67 kD).
- after two-dimensional electrophoresis of the isoelectric focusing type, followed by SDS-polyacrylamide gel electrophoresis, it separates into the following three antigenic proteins:
vi) a fraction whose apparent average molecular weight is approximately 210 kD, and whose pHi varies from approximately 5.85 to 6.85,
vii) a fraction whose apparent average molecular weight varies from approximately 130 to 90 kD, and whose pHi varies from approximately 5.85 to 7.35,
- a fraction whose apparent average molecular weight varies from approximately 120 to 67 kD, and whose pHi varies from approximately 5 to 7.5.
b) any one of the fractions i) to viii) from above-defined antigen a);
c) a protein antigen capable of being extracted from human epidermis, and which, after two-dimensional electrophoresis of the isoelectric focusing type, followed by SDS-polyacrylamide gel electrophoresis, presents an apparent average molecular weight of approximately 40 kD, and whose pHi varies from approximately 5.8 to 7.4.

2. Use of an antigen selected among:
- an antigen according to claim 1;
- human profilaggrin or filaggrin;
for the *in vitro* diagnosis of rheumatoid arthritis

3. A method for the in vitro diagnosis of rheumatoid arthritis in a biological sample, **characterized in that** it comprises at least the following steps:
- bringing said biological sample into contact with at least one antigen according to claim 1, or with a preparation of human profilaggrin or filaggrin optionally labelled, or alternatively with a conjugate thereof with a carrier molecule, under conditions that permit the formation of an immunological complex with the autoantibodies specific of RA which may be present in said sample;
- detecting said immunological complex.

4. A kit for the in vitro diagnosis of rheumatoid arthritis from a biological sample, **characterized in that** it comprises:
- at least one antigen according to claim 1 or at least one preparation of filaggrin or profilaggrin, optionally labelled, or conjugated with a carrier molecule;
- reagents to make up a medium suited to the immunological reaction with the autoantibodies which may be present in said biological sample;
- one or more reagents for detection of the immunological complex formed;
- if necessary, reference samples, such as negative serum/sera and/or positive serum/sera.

5. A method for purifying the autoantibodies specifically present in patients suffering from rheumatoid arthritis, **characterized in that** it comprises at least the following steps:
- bringing a biological fluid of a patient suffering from rheumatoid arthritis into contact with at least one antigen according to claim 1, or with human profilaggrin or filaggrin, under conditions that permit the formation of immunological complexes with the autoantibodies present in said biological fluid,
- isolating said complexes from the reaction medium and/or separating the autoantibodies from the said immunological complexes.

6. A method for preparing antibodies, **characterized in that** at least one antigen according to claim 1 is used as an immunogen.

7. A method for preparing antibodies **characterized in that** human profilaggrin or filaggrin is used as an immunization antigen, and **in that** the antibodies that also recognize an antigen from rat oesophageal epithelium, as defined in claim 1, are collected.

8. A method for preparing anti-idiotype antibodies, which method is **characterized in that** it comprises a step during which an animal is immunized with a monoclonal or polyclonal antibody obtained by a method according to claim 7.

9. A polyclonal antibodies preparation, **characterized in that** it is obtainable by the method according to claim 6.

10. An antibody preparation, **characterized in that** it is obtainable by the method according to claim 7.
